# EUROPEAN PATENT SPECIFICATION

(11) **EP 1 532 438 B1**
(45) Date of publication and mention of the grant of the patent: **12.05.2010**
(21) Application number: 03727020.4
(22) Date of filing: 13.06.2003
(51) Int. Cl.: G01N 33/48

(54) **OLIGOSACCHARIDE BIOMARKERS FOR MUCOPOLYSACCHARIDOSES AND OTHER RELATED DISORDERS**
OLIGOSACCHARID-BIOMARKER FÜR MUCOPOLYSACCHARIDOSEN UND ANDERE VERWANDTE STÖRUNGEN
BIOMARQUEURS OLIGOSACCHARIDES POUR LA MUCOPOLYSACCHARIDOSE ET D'AUTRES TROUBLES ASSOCIES

(30) Priority: 14.06.2002 AU PS293002
(43) Date of publication of application: 25.05.2005
(60) Divisional application: 09156934.3
(73) Proprietor: WOMEN'S AND CHILDREN'S HOSPITAL, North Adelaide, S.A. 5006 (AU)
(72) Inventor: MEIKLE, Peter, John, Redwood Park, S.A. 5097 (AU); FULLER, Maria, Prospect, S.A. 5082 (AU); RAMSEY, Steve, Lewis, Young, ACT 2594 (AU); RANIERI, Enzo, Woodville, S.A. 5011 (AU); HOPWOOD, John, Joseph, Stonyfell, S.A. 5066 (AU)
(74) Representative: Gates, Marie Christina Esther
(86) International application number: PCT/AU2003/000731
(87) International publication number: WO 2003/106997

(56) References cited:
- WO-A-03/048784
- WO-A2-01/94941
- RU-C2- 2 196 988
- US-A- 5 310 646
- LEISTNER SANDRA ET AL: "A useful routine for biochemical detection and diagnosis of mucopolysaccharidoses" GENETICS AND MOLECULAR BIOLOGY, [Online] vol. 21, no. 1, March 1998 (1998-03), XP002386981 ISSN: 1415-4757 Retrieved from the Internet: URL:www.scielo.br/scielo.php?script=sci_ar ttext&pid=S1415-47571998000100028> [retrieved on 2006-06-23]
- YANG HYUN OK ET AL: "Preparation and structural determination of dermatan sulfate-derived oligosaccharides" GLYCOBIOLOGY, vol. 10, no. 10, October 2000 (2000-10), pages 1033-1040, XP002386984 ISSN: 0959-6658
- DASGUPTA F ET AL: "Synthesis of 7-O-(2-deoxy-2-sulfamido-alpha-d-glucopyra nosyl)-4-methy lcoumarin sodium salt: a fluorogenic substrate for sulfamidase" CARBOHYDRATE RESEARCH, ELSEVIER SCIENTIFIC PUBLISHING COMPANY. AMSTERDAM, NL, vol. 337, no. 11, 5 June 2002 (2002-06-05), pages 1055-1058, XP004367095 ISSN: 0008-6215
- STARR C M ET AL: "Fluorophore-assisted carbohydrate electrophoresis in the separation, analysis, and sequencing of carbohydrates" JOURNAL OF CHROMATOGRAPHY A, ELSEVIER, AMSTERDAM, NL, vol. 720, no. 1, 12 January 1996 (1996-01-12), pages 295-321, XP004038366 ISSN: 0021-9673
- MEIKLE P J ET AL: "NEWBORN SCREENING FOR LYSOSOMAL STORAGE DISORDERS" SOUTHEAST ASIAN JOURNAL OF TROPICAL MEDICINE AND PUBLIC HEALTH, REGIONAL TROPICAL MEDICINE AND PUBLIC HEALTH, TH, vol. 30, no. SUPPL 2, 1999, pages 104-110, XP009065499 ISSN: 0125-1562
- RAMSAY S.L. ET AL.: 'Determination of monosaccharides and dissacharides in mucopolysaccharidoses patents by electrospray ionisation spectrometry' MOLECULAR GENETICS AND METABOLISM vol. 78, no. 3, 2003, pages 193 - 204, XP008045687
- ROZAKLIS T. ET AL.: 'Determination of oligosaccharides in pompe disease by electrospray ionization tandem mass spectrometry' CLINICAL CHEMISTRY vol. 48, no. 1, 2002, pages 131 - 139, XP008045690
- PITT J.J., GORMAN J.J.: 'Oligosaccharide characterization and quantitation using 1-phenyl-3-methyl-5-pyrazolone derivatization and matrix-assisted laser desorption/ionization time-of-flight mass spectrometry' ANALYTICAL BIOCHEMISTRY vol. 248, 1997, pages 63 - 75, XP008045705
- HOPWOOD J.J., HARRISON J.R.: 'High resolution electrophoresis of urinary glycosaminoglycans: and improved screening test for the mucopolysaccharidoses' ANALYTICAL BIOCHEMISTRY vol. 119, 1982, pages 120 - 127, XP008045692
- BYERS S. ET AL.: 'Glycosaminoglycan accumulation and excretion in the mucopolysaccharidoses: characterization and basis of a diagnostic test for MPS' MOLECULAR GENETICS AND METABOLISM vol. 65, 1998, pages 282 - 290, XP008045704
- HOPWOOD J.J., ELLIOTT H.: 'Urinary excretion of sulphated N-acetylhexosamines in patients with various mucopolysaccharidoses' BIOCHEM. J. vol. 229, 1985, pages 579 - 586, XP008045694
- DESAIRE H., LEARY J.A.: 'Detection of the sulfated disaccharides in chondroitin sulkfate by electrospray tandem mass spectrometry' JOURNAL OF THE AMERICAN SOCIETY FOR MASS SPECTROMETRY vol. 11, 2000, pages 916 - 920, XP004212319
- SWEETMAN L.: 'Newborn screening by tandem mass spectrometry: gaining experience' CLINICAL CHEMISTRY vol. 47, no. 11, 2001, pages 1937 - 1938, XP003003473
- PACKER N.H. ET AL.: 'A general approach to desalting oligosaccharides released from glycoproteins' GLYCOCONJUGATE JOURNAL vol. 15, 1998, pages 737 - 747, XP008045703
- GERBER S.A. ET AL.: 'Direct profiling of multiple enzyme activities in human cell lysates by affinity chromatography/electrospray ionization mass spectrometry: application to clinical enzymology' ANALYTICAL CHEMISTRY vol. 73, no. 8, 2001, pages 1651 - 1657, XP008045697
- FLUHARTY A.L.: 'The mucopolysaccharidoses: a synergism between clinical and basic investigation' THE JOURNAL OF INVESTIGATIVE DERMATOLOGY vol. 79, 1982, pages 38S - 44S, XP008045698
- WHITFIELD P. ET AL.: 'GM1-gangliosidosis in cross-bred dog confirmed by detection of GM1-ganglioside using electrospray ionisation-tandem mass spectrometry' ACTA NEUROPATHOL. vol. 100, 2000, pages 409 - 414, XP008045946
- WHITFIELD P D; SHARP P; MEIKLE P J; HOPWOOD J J: "Characterisation of ganglioside storage in mucopolysaccharidosis IIIA by tandem mass spectrometry" JOURNAL OF INHERITED METABOLIC DISEASE, vol. 23, July 2007 (2007-07), page 237,
- RANIERI E; RAMSAY S; BYERS S; TURNBULL J; HOPWOOD J J: "Nature of gags stored in mucopolysaccharidosis type VI" JOURNAL OF INHERITED METABOLIC DISEASE, vol. 23, July 2000 (2000-07), page 239,
- CHESTER M A; LENNARTSON G; LUNDBLAD A; LUNDSTEN J; NORDÉN N E; SJÖBLAD S; SVENSSON S; OCKERMAN P A: "Isolation and characterization of oligosaccharides from urine of patients with abnormal glycoconjugate metabolism" MONOGRAPHS IN HUMAN GENETICS, vol. 10, 1978, pages 2-6, SWITZERLAND

## Description

### BACKGROUND:

The present invention is generally related to diagnosing a pre - clinical status of mucopolysaccharidoses ("MPS") and related diseases. More particularly, this invention pertains to methods for identifying and quantitating oligosaccharides present in a biological fluid or tissue of a patient for use as biochemical markers ("biomarkers") for the diagnosis, characterization, monitoring, and clinical management of MPS.

Lysosomal storage disorders ("LSD") represent a group of over 40 distinct genetic diseases that generally affect young children. Individuals that are affected with a LSD present a wide range of clinical symptoms depending upon the specific disorder and the particular genotype involved. The clinical symptoms associated with LSD can have a devastating impact on both the child and the family of affected individuals. For example, central nervous system dysfunction, behavioral problems, and severe mental retardation are characteristic of many LSD. In a specific LSD group called MPS, other clinical symptoms may include skeletal abnormalities, organomegaly, corneal clouding, and dysmorphic features. Patients are usually born without visible clinical features of MPS, but can develop progressive clinical involvement. In severe cases, the affected children require constant medical management but still often die before adolescence.

The significance of LSD to health care becomes obvious when comparing the group incidence rate for LSD (1:5,000 births) to the group incidence rate of other well-known and intensively studied genetic disorders such as phenylketonuria (1:14,000) and cystic fibrosis (1:2,500) (figures reflect incidence rates for Caucasian populations). MPS represent a major group of LSD and have a combined incidence of 1:22,000 births in Australia. There are six types of MPS that are grouped as follows: (1) MPS I (Hurler or Scheie syndrome) results from a deficiency of α-L-iduronidase and leads to the lysosomal storage of the glycosaminoglycans ("GAG"), dermatan sulfate, and heparan sulfate; (2) MPS II (Hunter syndrome) results from a deficiency of iduronate-2-sulfatase and leads to the same GAG stored in lysosomes as found with MPS I; (3) MPS III (Sanfilippo syndrome) has four sub-types that all result in the storage of the one GAG, heparan sulfate, however, MPS IIIA, MPS IIIB, MPS IIIC and MPS IIID result from deficiencies of sulfamidase, α-N-acetylglucosaminidase, glucosamine acetyl-CoA: N-acetyltransferase and glucosamine-6-sulfatase respectively; 4) MPS IV (Morquio syndrome) has two sub-types, MPS IVA and IVB, both with lysosomal storage of the GAG keratan sulfate that results from a deficiency of N-acetylgalactosamine-6-sulfatase and β-galactosidase, respectively; (5) MPS VI (Maroteaux Lamy syndrome) results from lysosomal storage of the GAG dermatan sulfate due to a deficiency of N-acetylgalactosamine-4-sulfatase; and (6) MPS VII (Sly syndrome) resulting from a deficiency of β-glucuronidase and the lysosomal storage of dermatan sulfate and heparan sulfate.

There has been considerable progress in the diagnosis of LSD over the past 20 years. For example, the development and introduction of chromatographic-based urine screens for MPS and oligosaccharidoses has facilitated screening of clinically selected patients for these disorders. Following a clinical index of suspicion for MPS and certain oligosaccharidoses disorders, the next stage of diagnosis involves a urine screen, wherein a "positive" urine screen is then followed by specific enzymatic analysis. Although the screening methods are simple to perform, they are relatively labor-intensive and often require experience to accurately interpret results. Consequently, chromatographic-based screening tests for LSD are not used in some centers. Furthermore, these screens are not amenable to automation, which has further limited their utilization in screening strategies for newborns.

The production of specific substrates and antibody capture assays has made the enzymatic analyses for a specific LSD more accurate. However, many of these assays are still time-consuming, invasive, complex, and require cultured cells or tissue biopsies, which tends to make such assays inconvenient and expensive. As a result, testing for LSD is often not a first line strategy for an affected child with early stage symptoms. A clinical diagnosis of a LSD often requires multiple visits to a range of specialists, which can take months or even years. This long process is extremely stressful on the patient and family. Therefore, there is a need for the development of fast, accurate and economical screens for early diagnosis of LSD.

New therapies for many LSD have also changed the requirements for early diagnosis. For example, the efficacy of many of the proposed therapies will rely heavily upon early detection and treatment of the disease. Ideally, treatment should begin before the onset of irreversible pathologies and newborn screening for LSD would certainly provide early detection. However, if newborn pre-symptomatic diagnosis were realized, several concerns relating to patient-, disease-, and therapy-management would become key issues. For instance, the wide clinical spectrum displayed in many LSD will make proper selection of therapy difficult without additional information on the disease phenotype and the rate of disease progression. Furthermore, without a detailed description of the level of clinical severity in a LSD patient, establishing a prescription for drug or enzyme replacement therapy ("ERT") will be uncertain, and potentially hazardous to the patient. Therefore, methods to monitor disease progression, determine the particular phenotype, and monitor the effects of therapy in both the pre-clinically diagnosed and the clinically diagnosed individuals are also required.

Even after an individual begins to present the clinical symptoms of a LSD, the actual clinical diagnosis of the disease is still a complex process. For example, tests involving a range of assays must be performed on urine, blood and, in some disorders, skin fibroblasts. These assays are time consuming, expensive, and invasive, which makes them unsuitable for mass screening applications in newborns. For that reason, there is a need in the art to identify better biomarkers for the LSD. These biomarkers have application in the development of newborn screening programs, as well as the potential to address a number of the other issues highlighted above. Newborn screening for LSD promises to provide early detection of the disease, but all newborns must be screened in order to detect the disease in those affected. Patients having a family history of LSD have a justified reason to perform a pre-symptomatic screen for a LSD. However, the cost of a pre-symptomatic detection of the LSD in individuals not having a family history may not be justified economically. Therefore, it is essential that any LSD screening process be economically feasible such that all newborns can be screened.

There are several published papers describing methods for detecting and monitoring specific oligosaccharide biomarkers found in MPS and oligosaccharidoses patients. For example: In 1980, Kimura A, Hayashi S, Tsurumi K published a paper in Tohoku J Exp Med 131(3):241-7, entitled "Chemical Structure of Urinary Dermatan Sulfate Excreted by a Patient with the Hunter Syndrome." This paper described how the chemical structure of dermatan sulfate ("DS") in the urine of a patient the Hunter syndrome was studied through the analysis of disaccharide units that were derived from the urinary DS by digestion with chondroitinase ABC and separated on a Dowex 1 column. The DS was basically composed of repeating disaccharide units of iduronyl N-acetylgalactosamine-4-sulfate. About 90% of the excess sulfate was linked to the iduronate residues as an additional sulfate group in the unit. N-Acetylgalactosamine-6-sulfate and N-acetylgalactosamine-4,6-disulfate residues were minor components. No non-sulfated disaccharide unit was detected in the digestion products. Only sulfoiduronate residues were found as the non-reducing terminal sugar of the DS molecules, consistent with the lack of iduronosulfate sulfatase in this disease.

In 1981, Koseki M, Ino S, Kimura A, Tsurumi K published a paper in Tohoku J Exp Med 135(4):431-9, entitled "Abnormal Urinary Excretion in Sialoglycoconjugates in Patients with Mucopolysaccharidosis." Abnormal urinary excretion of sialoglycoconjugates was observed in four patients with MPS. Urinary sialoglycoconjugates were fractionated into 8 fractions by sequential gel filtration on Sephadex G-25, G-50 and by Dowex 1 ion-exchange chromatography. The comparison of the amounts of these fractions indicated that the fraction rich in mannose and glucosamine contents contributed to the increased urinary excretion of sialoglycoconjugates in patients with MPS. The major component of this fraction was disialyl-oroso-N-octaose which was a representative oligosaccharide side chain of glycoproteins with an Asn-N acetylglucosamine (GlcNAc) linkage. Although not wanting to be bound by theory, this abnormality is the secondary lesion of MPS, but it is conceivable that the disturbed metabolism of sialoglycoprotein is closely related to the pathogenesis of these diseases.

In 1983 Purkiss P, Gibbs DA, Watts RW published a paper in Clin. Chim. Acta. 131(1-2):109-21, entitled, "Studies on the Composition of Urinary Glycosaminoglycans and Oligosaccharides in Patients with Mucopolysaccharidoses who were Receiving Fibroblast Transplants." This communication reports studies on the composition of the urinary GAG and oligosaccharides in MPS patients that were being treated by fibroblast transplantation. The urinary GAG were precipitated with 9-aminoacridine, the oligosaccharides remaining in solution. Both fractions were further subfractionated by gel filtration. The GAG subfractions were examined for their content of iduronic acid, glucuronic acid, galactosamine and glucosamine. Although not wanting to be bound by theory, no changes were found in these parameters in a patient who had been treated by repeated fibroblast transplantations over the course of 4 1/2 years. The amino sugar composition of the oligosaccharide fraction was examined and shown to be unchanged. Additionally, no changes were found in the degree of sulfation of the urinary GAG specifically related to the transplant in four patients with Hurler disease and two with Hunter disease. The authors concluded that fibroblast transplantation does not produce detectable changes in the carbohydrate content or degree of sulfation of the urinary GAG and oligosaccharides.

In 1984, Elliott H, Hopwood JJ., published a paper in Anal. Biochem. 138(1):205-9, entitled "Detection of the Sanfilippo D Syndrome by the Use of a Radiolabeled Monosaccharide Sulfate as the Substrate for the Estimation of N-acetylglucosamine-6-sulfate sulfatase." This paper discussed how N-acetylglucosamine-6-sulfate sulfatase activity was assayed by incubation of the radiolabeled monosaccharide N-acetylglucosamine [1-14C]6-sulfate (GlcNAc6S) with homogenates of leukocytes and cultured skin fibroblasts and concentrates of urine derived from normal individuals, patients affected with N-acetylglucosamine-6-sulfate sulfatase deficiency (Sanfilippo D syndrome, MPS IIID), and patients affected with other MPS. The assay clearly distinguished affected homozygotes from normal controls and other MPS types. The level of enzymatic activity toward GlcNAc6S was compared with that toward a sulfated disaccharide and a sulfated trisaccharide prepared from heparin. The disaccharide was desulfated at the same rate as the monosaccharide and the trisaccharide at 30 times that of the monosaccharide. Sulfatase activity toward glucose 6-sulfate and N-acetylmannosamine-6-sulfate was not detected. Sulfatase activity in fibroblast homogenates with GlcNAc6S exhibited a pH optimum at pH 6.5, an apparent Km of 330 µmol/liter, and inhibition by both sulfate and phosphate ions. The use of radiolabeled GlcNAc6S substrate for the assay of N-acetylglucosamine-6-sulfate sulfatase in leukocytes and skin fibroblasts for the routine enzymatic detection of the Sanfilippo D syndrome is recommended.

In 1984, Kimura A, Hayashi S, Koseki M, Kochi H, Tsurumi K., published a paper in Tohoku. J. Exp. Med. 144(3):227-36, entitled "Fractionation and Characterization of Urinary Heparan Sulfate Excreted by Patients with Sanfilippo Syndrome". This paper discussed how the urinary heparan sulfates ("HS") from two siblings with MPS III-B were fractionated by chromatography with Dowex 1 and Sephadex G-50. Their Mr ranged from 1600 to 8000, and 95% of them were included in the region less than 5,000. Fractions with lower Mr contained larger amounts of O-and N-sulfates. The chemical analysis and deaminative cleavage of HS suggested that an intact HS molecule was composed of some blocks rich in GlcNAc and glucuronic acid (GlcUA) and other blocks rich in glucosamine-N-sulfate (GlcNS), iduronic acid (IdUA) and O-sulfate. GlcNAc-UA-GlcNS-UA-GlcNAc-UA-GlcNAc was found to be a major oligosaccharide of HS with Mr less than 1800. Trisaccharides, GlcNAc-GlcUA-anhydro-mannose (anMan) and GlcNAc-IdUA-anMan, were released from the non-reducing end of HS-oligosaccharides by deaminative cleavage. They carried 0-3 moles of ester sulfate. GlcNAc-IdUA-anMan was more sulfated than the other. The release of significant amounts of nonsulfated trisaccharide conform to the enzyme defect in this disease. Urinary HS obtained from another patient with MPS III were examined by the same way. Although the patient was not examined enzymatically, the structure of urinary GAG suggested a defect of alpha-N-acetylglucosaminidase in the patient.

In 1984, Kodama C, Ototani N, Isemura M, Yosizawa Z, published a paper in J. Biochem. (Tokyo) 96(4):1283-7, entitled "High-Performance Liquid Chromatography of Ppyridylamino Derivatives of Unsaturated Disaccharides Produced from Chondroitin Sulfate Isomers by Chondroitinases." This paper discusses how a sensitive method was developed for the separation and quantitation of four unsaturated disaccharides (delta Di-0S, delta Di-4S, delta Di-6S, and delta Di-diS) by high performance liquid chromatography. The unsaturated disaccharides were coupled with a fluorescent compound, 2-aminopyridine. Complete separation of the resulting pyridylamino derivatives was achieved on a column of muBondapak-C18 with 8 mM KH₂PO₄-Na₂HPO₄ (pH 6.0)/methanol (30/l, by volume) as a mobile phase. There was a linear relationship between the fluorescence emission (peak height), and the amount of each authentic disaccharide used for the coupling reaction. This method was applied to analyze commercially available chondroitin sulfates A and C, DS, and urinary GAG obtained from patients with MPS after digestion with chondroitinases. The data indicated that the present method is useful for the separation and quantitation of nmol-pmol levels of the unsaturated disaccharides produced from chondroitin sulfate isomers by chondroitinases and can be used for their structural characterization.

In 1985, Hopwood JJ, Elliott H., published a paper in Biochem. J. 229(3):579-86, entitled "Urinary Excretion of Sulphated N-acetylhexosamines in Patients with Various Mucopolysaccharidoses." Sulfated N-acetylhexosamines were isolated from human urine and tentatively identified as N-acetylglucosamine 6-sulfate (GlcNAc6S), N-acetylgalactosamine 6-sulfate (GalNAc6S), N-acetylgalactosamine 4-sulfate (GalNAc4S) and N-acetylgalactosamine 4,6-disulfate (GalNAc4,6diS). Urine from MPS-IIID, -IVA and -VI patients compared with that from normal individuals contains elevated levels of GlcNAc6S (380-fold), GalNAc6S (180-fold) and GalNAc4S (420-fold) respectively. Urine from MPS-VI patients also contain more than 600 times the normal level of GalNAc4,6diS. Urine from a mucolipidosis-Type-II and a multiple-sulfatase-deficient patient, and, in general, all MPS patients studied, contain at least 5-10-fold elevations of sulfated N-acetylhexosamines over the levels detected in urine from normal controls and a alpha-mannosidosis patient. Urine from patients with clinically mild phenotypes contains less sulfated N-acetylhexosamines than isolated from urine of clinically severe MPS patients. The source of the four sulfated N-acetylhexosamines is not known. However, incubation of a series of oligosaccharide substrates, derived from keratan sulfate and chondroitin 6-sulfate and containing non-reducing-end beta-linked 6-sulfated N-acetylhexosamine residues, with homogenates of cultured human skin fibroblasts has indirectly been shown to release GlcNAc6S and GalNAc6S respectively. Release of GalNAc4S could not be demonstrated in similar incubations of oligosaccharide substrates derived from chondroitin 4-sulfate and containing non-reducing-end beta-linked GalNAc4S residues. We propose that some, if not all, of the sulfated N-acetylhexosamine present in human urine is derived from the action of beta-N-acetylhexosaminidase on sulfated GlcNAc or GalNAc residues β-linked at the non-reducing end of keratan sulfate, dermatan sulfate or chondroitin sulfate.

In 1995, Murata K, Murata A, Yoshida K., published a paper in J. Chromatogr. B Biomed. Appl. 670(1):3-10, entitled "High-Performance Liquid Chromatographic Identification of Eight Constitutional Disaccharides from Heparan Sulfate Isomers Digested with Heparitinases." This paper showed identification with specific heparan sulfate-lyases, heparitinase I and heparinase of the constitutional unsaturated disaccharide (delta Di-SHS) derived from HS isomers and heparin was achieved using high-performance liquid chromatography ("HPLC") with a sulfonated styrene-divinylbenzene copolymer. Eight delta Di-SHS products derived from HS isomers were identified. Enzymatic digestion with heparitinase I and heparinase converts heterogeneous sulfated HS isomers and heparin into different delta Di-SHS. The practical application of these enzymes was examined using specific enzymes and HPLC. In a patient with Hurler syndrome, eight individual delta Di-SHS were identified in urinary HS isomers.

In 1996, Toma L, Dietrich CP, Nader HB., published a paper in Lab Invest., 75(6):771-81 entitled "Differences in the Non-reducing Ends of Heparan Sulfates Excreted by Patients with Mucopolysaccharidoses Revealed by Bacterial Heparitinases: a New Tool for Structural Studies and Differential Diagnosis of Sanfilippo's and Hunter's Syndromes." This paper discussed enzymatic and chemical analyses of the structures of HS excreted in the urine by patients with Sanfilippo's and Hunter's syndromes revealed that their non-reducing ends differ from each other and reflect the enzyme deficiency of the syndromes. The heparan sulfates from the different syndromes were treated with heparitinase II, crude enzyme extracts from Flavobacterium heparinum, and nitrous acid degradation. The HS from patients with Sanfilippo A (deficient in heparan N-sulfatase) and Sanfilippo B (deficient in alpha-N-acetylglucosaminidase) were degraded with heparitinase II producing, besides unsaturated disaccharides, substantial amounts of glucosamine N-sulfate and N-acetylglucosamine, respectively. The HS from patients with Hunter's syndrome (deficient in iduronate sulfatase) were degraded by heparitinase II or crude enzyme extracts to several products, including two saturated disaccharides containing a sulfated uronic acid at their non-reducing ends. The HS from patients with Sanfilippo's C syndrome (deficient in acetyl Co-A: alpha-glucosaminide acetyltransferase) produced, by action of heparitinase II, among other products, two sulfated trisaccharides containing glucosamine with a nonsubstituted amino group. In addition to providing a new tool for the differential diagnosis of the MPS, these results bring new insights into the specificity of the heparitinases from flavobacterium heparinum.

In 1998 Byers, S. Rozaklis, T. Brumfield, L. K. Ranieri, E. and Hopwood, J. J. published a paper in Mol Genet Metab. 1998 Dec;65(4):282-90. entitled "Glycosaminoglycan accumulation and excretion in the mucopolysaccharidoses: characterization and basis of a diagnostic test for MPS". In this study the authors used a combination of anion-exchange chromatography and 30-40% gradient polyacrylamide gel electrophoresis (gradient-PAGE) to purify and characterize urinary GAG from various MPS. The urinary GAG from the different MPS displayed distinct patterns on gradient-PAGE and further confirmation of MPS types and subtypes was demonstrated by an electrophoretic shift in the banding pattern after digestion with the appropriate MPS enzyme. They reported that each of the MPS accumulates a unique spectrum of GAG with a non-reducing terminal consisting of the substrate specific for the deficient enzyme in that particular MPS disorder. The absolute correlation of the non-reducing terminal structure with a particular MPS and the availability of recombinant lysosomal enzymes provide the means for a rapid and accurate diagnosis of individual MPS.

In 2003, after the filing of the Australian provisional patent SN PS2930, Ramsay SL, Meikle PJ, Hopwood JJ, published a paper in Mol. Genet. Metab. 78(3): 193-204, entitled "Determination of Monosaccharides and Disaccharides in Mucopolysaccharidoses Patients by Electrospray Ionization Mass Spectrometry." This paper discusses how the MPS are a group of LSD characterized by the storage of GAG. With the exception of Hunters syndrome (MPS II), which is X-linked, they are autosomal recessively inherited resulting in a defect in any one of 10 lysosomal enzymes needed to catabolise GAG. The type and size of the GAG stored in lysosomes are determined by the particular enzyme deficiency. These GAG elevations are subsequently observed in tissue, circulation, and urine. A method has been developed for the derivatization and quantification of sulfated N-acetylhexosamine-containing mono- and disaccharides from patient samples by electrospray ionisation tandem mass spectrometry. Urine from most MPS types had significant increases in disulfated and monosulfated N-acetylhexosamines (GalNAc4,6S, GalNAc6S, GalNAc4S, or GlcNAc6S) and monosulfated N-acetylhexosamine-uronic acid disaccharides (GalNAc6S-UA, GalNAc4S-UA, or GlcNAc6S-UA). Analysis of plasma and dried blood spots on filter paper collected from MPS patients showed elevations of total monosulfated N-acetylhexosamines but less than that seen in urine. Urine samples from bone marrow transplant recipients, MPS IVA and MPS VI patients, showed decreases in HexNAcS, HexNAcS(2)/GalNAc4,6S, and HexNAcS-UA post-transplant. This decrease correlated with clinical improvement to levels comparable with those identified in patients with less severe phenotypes.
US Patent No. 5310464 describes a method for screening for mucopolythaccharide storage diseases in urine samples absorbed onto a porous sheet, followed by use of 1,n9n-dimethylmethylene blue dye and spectrophotometric analysis. Leistner and Giugliani, Genetics and Molecular Biology, Vol 21, p163 to 167 (1998) disclose a method for the biochemical detection and diagnosis of mucopolysaccharidoses in blood and urine samples involving cetyltrimetylammium bromide screening and toluidine blue spot tests. Yang Hyun Ok et al, Gylcobiology Volume 10, Pages 1033 to 1040 (2000) describes the preparation and structural determination of dermatan sulphate-derived oligosaccharides. Dasgupta et al " Carbohydrate Research, Elsever Publishing Company Amsterdam Vol 337, Pages 1055 to 1058 (2002) describes the synthesis of 7-*O*(2-deoxy-2-sulfamido-a-D-glucopyranosyl)-4-methyl coumarin sodium salt which is useful in the testing in the accuracy of heparin sulfamidase. Starr et al, J. Chromatography Vol 720, p295-321 (1996) describes fluorophore-assisted carbohydrate electrophoresis in the separation, analysis and sequencing of carbohydrates. The technique can be used for performing routine oligosaccharide profiling, monosaccharide analysis, and sequencing of a variety of carbohydrates. Meikle et al, Southeast Asian J. Tropical Medicine and Public Health, Regional Tropical Medicine and Public Health Vol 30, p104 to 110 (1999) describes the measurement of Lysosomal proteins in blood spots taken from Guthrie cards for newborn babies, for the detection of lysosomal storage disorders. Rozaklis et al, Clinical Chemistry, Vol 48, p131 to 139 (2002) describes a multiplexed immune-quantification assay of 11 lysosomal proteins for the identification of individuals with a lysosomal storage disorder. Hopwood and Harrison, Analytical Biochemistry Vol 119, pl20 to 127 (1982) describes an approved screening test for mucopolysacchridoses involving high resolution electrophoresis of urinary glycosaminoglycns. Byers et al, Molecular Genetics and Metabolism Volume 46, Pages 282 to 290 (1998) describes accumulation and excretion of glycosaminoglycan in mucopolysaccharidoses and their characterisation as the basis for a diagnostic test for mucopolysaccharidoses.

Accordingly, there is a need for the development of a fast, accurate and economical screen for early diagnosis of LSD, which is also amenable to automation. Furthermore, the screening methods should have the ability to monitor the effects of therapy in clinically-affected and clinically-unaffected individuals. Therefore, the identification of such diverse clinical biomarkers for LSD would have a significant impact on the development of a newborn screening programs, as well as the ability to address a number of the other issues associated with the early diagnosis and treatment of LSD. The present invention provides methods for detecting, quantitating, and monitoring specific oligosaccharide biomarkers found in MPS and oligosaccharidoses patients.

According to the present invention there is provided method for diagnosing a pre-clinical status, or a clinical status of a mucopolysaccharidoses (" MPS") disease in a target animal comprising:
(a) determining a target quantity of a target MPS biomarker from a target biological sample taken from the target animal; and
(b) comparing the target quantity to a reference quantity of a reference MPS biomarker; wherein
the target MPS biomarker is the same or equivalent to the reference MPS biomarker, and each of the target MPS and the reference biomarker is an oligosaccharide, wherein the oligosaccharide comprises a sulphated or unsulfated molecule having a sugar length from 1 to 12 residues,
the reference quantity is determined from a reference animal, or group of reference animals, having a known MPS clinical status; and
a deviation of the target quantity of the target MPS biomarker from the reference quantity of the reference MPS biomarker is a pre-clinical or clinical indication of the MPS disease, an indication of a progression of the MPS disease, or an indication of a regression of the MPS disease, wherein determining the target quantity comprises a mass spectrometric analysis.

In a preferred embodiment, the target MPS biomarker and the reference MPS biomarker are derivatized by reacting the MPS biomarkers with a derivatizing agent prior to determining the target quantity and reference quantity. The derivatizing agent may comprise 1-phenyl-3-methyl-5-pyrazolone ("PMP"). In a preferred embodiment, the target quantity and the reference quantity of the MPS oligosaccharide biomarkers are determined using a tandem mass spectrometry method. However, identification methods from other embodiments can be selected from a chromatographic assay, an immunoassay, liquid chromatography, anion exchange chromatography, size exclusion chromatography, MALDI-TOF mass spectrometry, mass spectrometry, or combination thereof.

In preferred embodiments, the MPS biomarkers correspond to specific oligosaccharides, wherein the oligosaccharide comprises a sulfated or unsulfated molecule having a sugar length ranging from 1 to 12 residues. These oligosaccharides comprise cleavage products of a GAG that are identified from the target biological sample, wherein the GAG is HS, DS, keratan sulfate, or chondroitin sulfate. In another preferred embodiment, the MPS biomarkers contained in a target biological sample are digested with a first functional enzymatic equivalent of a deficient enzyme that characterizes a particular MPS disease subtype. For example, the first functional enzymatic equivalent of the deficient enzyme in MPS-I comprises α-L-iduronidase. When the digesting step is used before determining the target quantity of the target MPS biomarker, the sensitivity for the quantification is improved.

In one embodiment, the MPS biomarker oligosaccharide is used to diagnose MPS in a target animal or patient by comparing the target quantity of the MPS biomarker oligosaccharide with a reference quantity of the same, or similar MPS biomarker oligosaccharide. For example, a target animal can be diagnosed as having the MPS disease when a range of target quantity values of the MPS biomarker oligosaccharides is greater than a range of values for the reference quantity oligosaccharides derived from animals not having a MPS disease. Additionally, the target quantity of MPS biomarker oligosaccharides is greater in the target animal having the MPS disease when compared to the reference quantity of MPS biomarkers oligosaccharides in the reference animal having a similar, but diminished phenotype of the MPS disease. Alternatively, the target quantity of MPS biomarker oligosaccharides is greater in a target animal having the MPS disease when compared to the same target animal having the MPS disease, but at a time after receiving an effective MPS therapy, wherein the effective MPS therapy comprises a bone marrow transplant ("BMT"), or MPS enzyme replacement therapy. A comparison of the target quantity to the reference quantity can be utilized to determine the appropriate type or the extent of MPS therapy.

In another specific embodiment, an internal standard is utilized to accurately determine the quantity of the target MPS biomarker or reference MPS biomarker. A deuterated N-acetylglucosamine-6-sulfate ("GlcNAc6S(*d3*)") was used as an internal standard in a preferred embodiment. Other internal standards comprise non-physiological oligosaccharides that are similar to the oligosaccharide being investigated. For example, a non-physiological oligosaccharide that is derived from a chondroitinase digestion of chondroitin sulfate, but having an unsaturated uronic acid at the non-reducing end.

The biological sample can be selected from urine, a cellular extract, blood, plasma, CSF or amniotic fluid, but urine is used in preferred embodiments. The method comprises MPS biomarkers that are selective for subgroups of MPS diseases, wherein the MPS disease is MPS-I, MPS-II, MPS-IIIA, MPS-IIIB, MPS-VI, MPS-IIIC, MPS-IIID, MPS-IV, or a combination thereof. The method can be used in a newborn for the diagnostic screening of the MPS disease.

A second aspect of the current invention also pertains to a method for diagnosing a preclinical status, or a clinical status, of a MPS disease in a target animal. The method comprises: derivatizing the MPS biomarker in a sample obtained from a target animal having a MPS biomarker with a derivatizing agent, or solution, to form a derivatized MPS biomarker; contacting the derivatized MPS biomarker with a solid phase extraction column; washing the solid phase extraction column with a washing solution; eluting the derivatized MPS biomarker from the solid phase extraction column with an elution solution; determining an eluted MPS biomarker quantity; and comparing the eluted MPS biomarker quantity with a reference MPS biomarker quantity. The reference MPS biomarker quantity is determined from a reference group animal, or group of reference animals, having a known MPS clinical status. For this method, the MPS biomarker is the same or equivalent to the reference MPS biomarker, and each of the MPS biomarker and the reference MPS biomarker is an oligosaccharide. Additionally, the MPS biomarker quantity and the reference MPS biomarker quantity are normalized, and in certain embodiments each quantity is normalized to creatinine or another oligosaccharide. A deviation of the eluted MPS biomarker quantity when compared to the reference MPS biomarker quantity is considered a preclinical or clinical indication of the MPS disease, a progression of the MPS disease, or a regression of the MPS disease.

The MPS biomarkers in the biological sample may also be lyophilized prior to the derivatization step, wherein a preferred derivatization solution comprises PMP. The MPS biomarkers correspond to specific oligosaccharides, wherein the oligosaccharide comprises a sulfated molecule having a sugar length ranging from 1 to 12 residues. These oligosaccharides comprises a cleavage product of a GAG that are identified from the target biological sample, wherein the GAG is HS, DS, keratan sulfate, or chondroitin sulfate. In another specific embodiment, the MPS biomarker oligosaccharides contained in a target biological sample are digested with a first functional enzymatic equivalent of a deficient enzyme that characterizes a particular MPS disease subtype. For example, the first functional enzymatic equivalent of the deficient enzyme in MPS-I comprises α-L-iduronidase. When the digesting step is used before determining the target quantity of the target MPS biomarker, the sensitivity for the quantification is improved.

A third aspect of the current invention is a kit used for diagnosing a preclinical status, or a clinical status of a MPS disease in a target animal. In a preferred embodiment, the kit comprises: an oligosaccharide derivatization agent used to alter the chemical composition a target MPS biomarker; an acid solution used to neutralize the derivatization agent following a derivatization reaction; an internal standard used to accurately determine the quantity of the target MPS biomarker; a solid phase extraction column used to purify a derivatized target MPS biomarker; a solid phase extraction column wash solution used to remove impurities from the solid phase extraction column having a bound target MPS biomarker; an oligosaccharide elution solution used to elute the target MPS biomarker from the solid phase extraction column; and a set of instructions for using the kit.

In one specific embodiment, the oligosaccharide derivatization solution comprises PMP. The preferred acid solution comprises formic acid. The derivatized oligosaccharide MPS biomarkers are absorbed onto a solid phase extraction column. The preferred column comprises a C18 reverse phase column, and the preferred column wash solution comprises CHCl₃. In order to remove the bound oligosaccharides from a bound column, the preferred elution solution comprising CH₃CN and formic acid is used.

Also included in the kit is the internal standard, wherein a GlcNAc6S(*d3*) is the preferred internal standard. However, a non-physiological oligosaccharide that is similar to the oligosaccharide being investigated can also be use as an internal standard. For example, the non-physiological oligosaccharide may be derived from a chondroitinase digestion of chondroitin sulfate having an unsaturated uronic acid at the non-reducing end.

### BRIEF DESCRIPTION OF THE DRAWINGS

Figure 1 shows the elution profile of uronic acid (UA) equivalents from fractionated MPS I and control urine from a Bio-Gel P2 column;

Figure 2 shows a mass spectra of a product ion scan having daughter ions produced from the parent ion, yielding information about their structure as well as providing suitable ion pairs for multiple reaction monitoring ("MRM");

Figure 3 shows oligosaccharides in MPS I urine, wherein fragmentation patterns were used to delineate structural composition and the residue on the non reducing end;

Figure 4 shows the mass spectra of tetrasaccharide (*m*/*z* 632) following recombinant enzyme digests;

Figure 5 shows a Q1 mass spectra characteristic of a non-reducing end of a trisaccharide and a tetrasaccharide isolated from MPS I urine in the absence (Fig 5A) and presence (Fig 5B) of α-L-iduronidase;

Figure 6 shows Q1 mass spectra of oligosaccharides found in the urine from a MPS II patient;

Figure 7 shows Q1 mass spectra of oligosaccharides found in the urine from a MPS II patient;

Figure 8 shows a list of average ratio values and standard deviation ("SD") of oligosaccharide signals to the internal standards values used for the MRM analysis of selected oligosaccharides;

Figure 9 shows the Mann Whitney U values for selected oligosaccharide analytes in MPS subgroups compared to 26 controls;

Figure 10 shows a box plot of the relative levels of 9 GAG derived oligosaccharides analyte in control individuals;

Figure 11 shows several box plots of the relative levels of 9 GAG derived oligosaccharides analyte from patients having different MPS sub-group disorders;

Figure 12 shows the relative concentrations of oligosaccharides in urine from control and MPS I patients;

Figure 13 shows the relative levels of oligosaccharides in skin fibroblasts (e.g. oligosaccharide/mg cell protein) from patents having the MPS I phenotype are higher than relative levels found in controls;

Figure 14 shows the levels of each MRM pair of selected oligosaccharides that were elevated in MPS I patients having various phenotypes when compared to a control or reference level;

Figure 15 shows the relative oligosaccharide levels in a MPS I patient before and after receiving a bone marrow transplant ("BMT") compared to control or reference values;

Figure 16 shows the relative levels of GAG derived oligosaccharides in urine from a MPS IVA affected individual before and after a bone marrow transplant;

Figure 17 shows the relative levels of GAG derived oligosaccharides in urine from a MPS VI affected individual before and after a bone marrow transplant;

Figure 18 shows a time course of the total urinary GAG for normal animals, MPS VI untreated animals, MPS VI low dose treated animals, and MPS VI high dose treated animals, wherein GAG was determined using the DMB dye binding assay and normalized to creatinine;

Figure 19 shows a time course for the concentration of the monosaccharide HexNAcS in urine collected from normal animals, MPS VI untreated animals, MPS VI low dose treated animals, and MPS VI high dose treated animals, wherein the concentration of the monosaccharide HexNAcS was measured using tandem mass spectrometry and normalized to creatinine;

Figure 20 shows a time course for the concentration of the disaccharide HexNAcS-UA in urine was collected from normal animals, MPS VI untreated animals, MPS VI low dose treated animals, and MPS VI high dose treated animals wherein HexNAcS-UA was measured using tandem mass spectrometry and normalized to creatinine;

Figure 21 shows a time course for the relative concentrations of the disaccharides HexNAcS-UA and HexNAc-UA in urine was collected from normal animals, MPS VI untreated animals, MPS VI low dose treated animals, and MPS VI high dose treated animals, wherein the relative concentrations of the disaccharides HexNAcS-UA and HexNAc-UA were measured using tandem mass spectrometry and are expressed as a ratio;

Figure 22 shows the concentration of the monosaccharide HexNAcS in blood collected from normal animals, MPS VI untreated animals, MPS VI low dose treated animals, and MPS VI high dose treated animals, wherein the concentration of the monosaccharide HexNAcS was determined using tandem mass spectrometry;

Figure 23 shows the monosaccharide and oligosaccharide levels in control and MPS I cell lysates before and after treatment with α-L-iduronidase;

Figure 24 shows a LC-MSMS identification of an oligosaccharide in a MPS patient before and after the oligosaccharide was treated with a recombinant N-acetylgalactosamine-4-sulfatase;

Figure 25 shows MRM ratios of oligosaccharides in skin fibroblasts post-confluence;

Figure 26 shows the MRM ratios of oligosaccharide levels following recombinant α-L-iduronidase (rhIDUA) addition.

**DETAILED DESCRIPTION OF THE PREFERRED EMBODIMENTS**

### Terms:

The term "a" or "an" as used herein in the specification may mean one or more. As used herein in the claim(s), when used in conjunction with the word "comprising", the words "a" or "an" may mean one or more than one. As used herein "another" may mean at least a second or more.

The term "animal"; "subject"; "individual"; or "patient" as used herein may be used interchangeably and refers to any species of the animal kingdom, including humans.

The term "clinical status", as used herein refers to patients that are being studied or treated by physicians for a MPS disorder.

The term "comprise", or variations such as "comprises" or "comprising", as used herein may be used to imply the inclusion of a stated element or integer or group of elements or integers, but not the exclusion of any other element or integer or group of elements or integers.

The term "mucopolysaccharidoses (MPS)", as used herein refers to a subgroup of lysosomal storage disorders ("LSD") characterized by the accumulation and storage of GAG within lysosomes.

The term "MPS biomarker", as used herein refers to an oligosaccharide that is detectable in a biological fluid from an individual. The quantity of this MPS biomarker is indicative of a clinical or preclinical status of a MPS disorder in an individual.

The term "MPS enzyme replacement therapy", as used herein refers to any drug or therapy that replaces a deficient or defective enzyme in a MPS patient.

The term "normalize", as used herein refers to bringing a target, reference, or other samples into conformity with a standard, pattern, model, etc. For example, in one embodiment, urine samples from a MPS patients and a non-MPS patients were normalized by using a 1µmol equivalent of creatinine from each sample.

The term "phenotype", as used herein refers to the manifest characteristics of an organism collectively, including anatomical and psychological traits, that result from both its heredity and its environment.

The term "preclinical status", as used herein refers to the period of a disease before any of the symptoms appear.

The term "reference quantity", as used herein refers a known, normalized amount of a MPS biomarker in a biological fluid. The reference quantity is determined from an animal, or group of animals having a defined clinical status, preclinical status, or phenotype of a MPS disease. The reference quantity may refer to a table compiled from various animals or groups of animals having correlations between relative amounts of MPS biomarkers in a biological fluid, and a known clinical status, preclinical status, or phenotype.

One common feature of LSD is the accumulation and storage of materials within lysosomes. It is generally recognized that the accumulation and storage of material in LSD affected individuals results in an increase in the number and the size of lysosomes within a cell from approximately 1% to as much as 50% of total cellular volume. In non-affected individuals, such materials are typically degraded into degradation products within the lysosome and then transported across the lysosomal membrane. The inventors have previously found that certain lysosomal proteins are present at elevated levels in the lysosomes from affected individuals (Meikle et al 1997; Hua et al 1998). These identified proteins can be useful biomarkers for an early diagnosis of all LSD. For example, the lysosome-associated membrane proteins ("LAMPs"), the saposins and α-glucosidase have been identified as useful biomarkers, and sensitive immunoquantification assays have been developed to monitor the level of these proteins. Although the determination of either LAMP-1 or LAMP-2 levels alone in an 'at-increased-risk' group will identify up to 65% of LSD affected individuals, the combination of a LAMP with one of the saposins increases identification of LSD affected individuals to approximately 85%.

A second characteristic feature of LSD is that the stored substrates are eventually released from cells either by exocytosis or as a result of membrane rupture following cell death. Consequently, the identification and quantification of specific substrates in various biological fluids (e.g. blood or urine) can confirm LSD in an individual. Furthermore, the quantification of specific substrates also resolves the specific type of disorder that is involved. The level of stored substrates correlates to disease severity which will be critical for the monitoring of clinical or pre-clinical progression and the effects of forthcoming therapy trials (i.e. animal and human).

In the MPS group of disorders, the primary storage material in lysosomes comprise GAG, in particular HS, DS, keratan sulfate and chondroitin sulfate. Because endoglycosidases are capable of cleaving GAG, the inventors determined that smaller oligosaccharide fragments are useful biomarkers for the MPS group of disorders. Thus, the inventors have identified that elevated levels of specific oligosaccharides (in particular, sulfated oligosaccharides) in a biological sample taken from an 'at risk' individual are indicative of MPS or a multiple sulfatase deficiency. The oligosaccharides of interest will generally comprise 1 to 12 sugar residues (i.e. they will range from monosaccharides to dodecasaccharides). Although not wanting to be bound by theory, these oligosaccharides are derived from the GAG storage substrates that accumulated in patients having these disorders.

Accordingly, determining the quantity of certain oligosaccharides (e.g., sulfated monosaccharides to dodecasaccharides) in a biological sample taken from an animal provides a screening or monitoring method for MPS or multiple sulfatase deficiencies. Although not wanting to be bound by theory, the elevated quantities of the oligosaccharides are indicative of the presence the disorder, and the formation of the oligosaccharides occur due to cleavage of one or more GAG. The oligosaccharides can be derived from one or more of the following GAG: HS; DS; keratan sulfate; or chondroitin sulfate, and any method wherein specific oligosaccharides can be identified (e.g. tandem mass spectrometry, mass spectrometry, liquid chromatography and/or immunoassay) will allow for the identification of and monitoring of MPS or multiple sulfatase deficiencies in an 'at risk' individual.

It is an object of the present invention that elevated levels oligosaccharides in biological samples can be utilized as neonatal screening markers or for monitoring progress of a patient being treated for a MPS. The subject may or may not have already been diagnosed with a MPS disorder (i.e. pre-clinical or clinical). The clinical condition of the subject may be monitored by these methods to determine the efficacy of a treatment regime (e.g. enzyme replacement therapy, gene therapy and/or dietary therapy). In addition, the invention may be better understood with reference to one or more of the following examples, which are representative of some of the embodiments of the invention.

### EXAMPLE 1

**Sample Preparation: Isolation of oligosaccharides from a biological sample:** The biological sample may comprise urine, blood, cells or any other biological sample that contains the oligosaccharides of interest. Because the collection of urine samples is an easy, non-invasive procedure, urine was used as the biological sample in some of the following examples. However, the utilization of urine as a biological sample is not intended to exclude other biological samples from being used for the determination of specific oligosaccharides. Furthermore, one skilled in the art will appreciate that some of the reagents used in the following examples can be varied to process different types of biological samples without deviating from the scope and spirit of the invention.

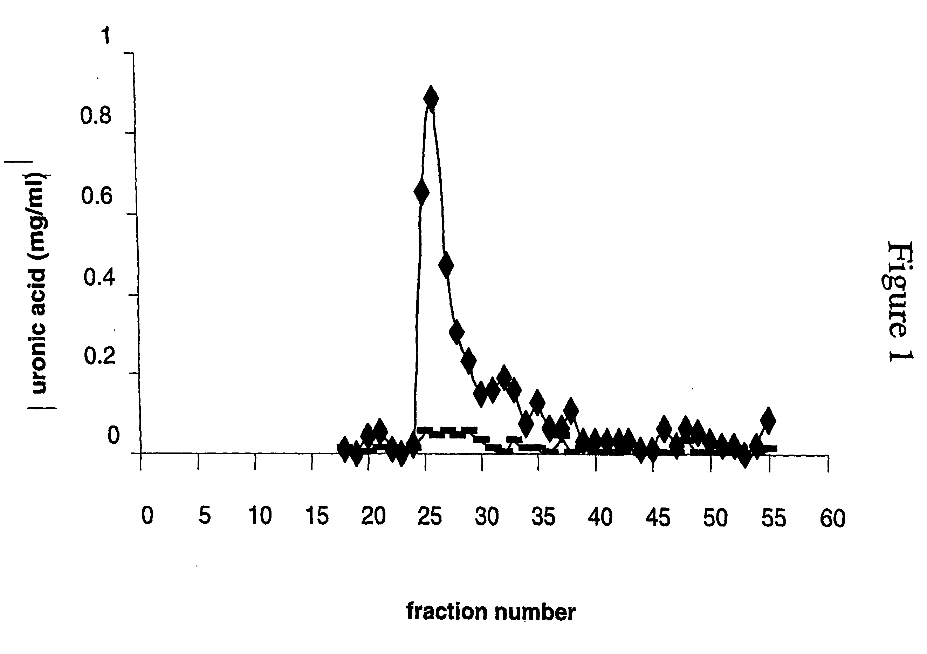

In some of the examples given below, the urine from patients was obtained with consent and processed as follows: To remove debris, 500ml of urine was collected and clarified by centrifugation at 2000 x g for 10 min. The clarified urine was then passed over a 50ml column of DEAE-Sephacel that had been previously equilibrated with 0.1M NaCOOCH₃ pH 5.0 under gravity. The DEAE-Sephacel column was then washed with 10 column volumes of the equilibration buffer. The urinary GAG were eluted with 0.1 M NaCOOCH₃ pH 5.0 containing 1.2 M NaCl. The eluate fractions from the DEAE-Sephacel column were assayed for uronic acid. The fractions containing substantial quantities uronic acid were pooled, lyophilized, and reconstituted in 4.0ml of water. The pooled GAG's were then fractionated on a Bio-Gel P2 column (1.5cm ID x 170cm length) that had been previously equilibrated in 0.5 M NH₄COOH. Fifty-five Bio-Gel P2 fractions of 4.0ml were collected and a second assay for uronic acid was performed. Figure 1 shows the elution profile of the oligosaccharides from a MPS I patient urine (solid diamonds) and a control urine (grey line).

Fractions 31 to 54 were subsequently derivatized for mass spectrometry as described below. However, one skilled in the art will appreciate that many reagents used for purifying GAG from biological samples may be utilized without deviating from the scope and spirit of the invention.

**Preparation of GlcNAc6S(*d3*):** The deuterated internal standard GlcNAc6S(*d3*) was prepared by selective N-acetylation of the glucosamine-6-sulfate (GlcN6S). The monosaccharide GlcN6S (25mg) was dissolved in anhydrous solutions of pyridine (700 µl), dimethyl formamide (700 µl) and methanol (50 µl) by sonication. The solution was stirred on ice and acetic anhydride(*d₆*) (2 x 20 µl) was added at 30 minute intervals. After one hour, the reaction was quenched with a 4% aqueous ammonia solution (500µl) and then placed under a stream of nitrogen to remove solvents. This step was repeated. The remaining residue was dissolved in water (500 µl) and loaded onto an anion exchange column (AG1-X8, H⁺ form, 100-200 mesh, 5 ml bed) and washed with deionized water (4 column volumes). The monosaccharide was eluted with LiCl (2.5 M) and the fractions monitored with the bicinchoninic acid microwell assay (BCA™, Pierce Chemical Company, Rockford Illinois). The fractions containing GlcNAc6S(*d3*) were pooled and desalted on a size exclusion column (50 x 1 cm) of P2 fine (Bio-Rad). The desalted fractions were pooled and lyophilized to produce 5.5 mg of a white solid (18.7 % yield). The purity of the GlcNAc6S(*d3*) was determined by comparison to the un deuterated GlcNAc6S in the mass spectrometer. A neutral loss (NL374) ESI/MSMS experiment showed that the GlcNAc6S(*d3*) compound was 83.95 % pure (w/w). However, one skilled in the art will appreciate that many reagents used to produce a deuterated internal standard GlcNAc6S(*d3*) may be utilized without deviating from the scope and spirit of the invention.

**Derivatization of oligosaccharides:** Samples of purified oligosaccharides, cell lysates and urine (0.5-1.0 µmol creatinine equivalents) were lyophilized prior to derivatization, and whole blood samples were dried onto filter paper (S&S 903, Schleicher & Schuell, Dassel, Germany) and 3mm punches were taken and derivatized directly. Each sample had 50-100 µL of derivatizing solution (250 mmol/L 1-phenyl-3-methyl-5-pyrazolone ("PMP"), 400 mmol/L NH₃, pH 9.1 containing 1 nmol sulfated disaccharide and 1 nmol GlcNAc6S(*d3*)) added, wherein the solution was vigorously stirred using a vortex mixer and then heated in an oven at about 70°C for 90 minutes. Samples were then acidified with a 2 fold molar excess of formic acid (50 µL, 800 mmol/L) and made up to 500 µL with water. Each sample was extracted with 500 µL of CHCl₃ to remove excess PMP and centrifuged (13,000xg, 5 minutes). Solid phase extraction columns (25 mg, C18) were primed with successive 1 ml washes of 100% CH₃CN, 50% CH₃CN/0.025%FA and water. The aqueous layer from each CHCl₃ extraction (400 µL) was applied to a primed C18 column and allowed to enter the solid phase completely. The column was washed with water (1 X 500 µL, followed by 2 X 1000 µL) and dried under vacuum (15 minutes) on a Supelco, Visiprep24 vacuum manifold (Sigma-Aldrich, St Louis, USA) or in a lyophilizing device (45 minutes), if in the 96-well format. Each dried C18 column was then washed with CHCl₃ (2 X 1000 µL) to remove any unincorporated PMP, and again dried thoroughly. Derivatized oligosaccharides were eluted from each C18 column with 50% CH₃CN / 0.025% formic acid in water (3 X 200 µL) and dried under a stream of N₂. Each sample was then reconstituted in 500 µL of 50% CH₃CN / 0.025% formic acid in water for injection into the mass spectrometer.

**Mass spectrometry** Oligosaccharide analysis was performed by ESI-MS/MS using a PE Sciex API 3000 triple-quadrupole mass spectrometer with an ionspray source and Analyst 1.1 data system (PE Sciex). Samples were either directly infused using a Harvard Apparatus pump at 10 µl/min or injected with a Gilson 233 autosampler at 50 µl/min using a carrying solvent of 50 % (v/v) acetonitrile/0.025 % (v/v) formic acid in water. Oligosaccharides were identified based on mass to charge ratios (*m*/*z*) in Q1 scans and further characterised using product ion scans in the negative ion mode. Quantification of these oligosaccharides was performed using multiple-reaction monitoring (MRM) in negative ion mode. Each MRM pair was monitored for 100 milliseconds at unit resolution. For each measurement 100 consecutive scans were averaged and relative concentration ratios were calculated by relating the peak heights of the PMP-oligosaccharides to the peak height of the internal standard.

**HPLC quantification.** Although mass spectroscopy is the preferred method to determine oligosaccharides of this invention, another aspect of the current invention involves HPLC quantification of the oligosaccharides derived from GAG's. For example, the oligosaccharides from a urine sample that have been derivatized with PMP, as described above, can also be separated by ion pairing-reverse phase HPLC. The derivatized oligosaccharides can be dissolved in water containing a suitable ion-pairing reagent (1 mM triethylamine) and injected onto a C18 reverse phase HPLC column. The sulfated oligosaccharides can be eluted from the column using a gradient of acetonitrile/water containing the ion pairing reagent (1 mM triethylamine) and quantified by absorbance at 260 nm. However, one skilled in the art will appreciate that many HPLC reagents and methods can be modified to determine the oligosaccharides of this invention without deviating from the scope and spirit of the invention.

**Immune-quantification assay.** Another preferred method of the detection and quantification of specific oligosaccharides utilizes monoclonal antibodies in an immune-quantification assay. For example, the oligosaccharide can be coupled to a protein carrier and used to coat the inside of a microtiter plate well. A monoclonal antibody directed against the oligosaccharide can be labeled with a suitable reporter molecule and added to the microtiter plate well. Additionally, a sample containing an oligosaccharide of interest can be added to the well, which results in a partial binding inhibition of the monoclonal antibody to the immobilized oligosaccharide on the well surface. A calibration curve, with known amounts of the oligosaccharide can then be used to quantify the level of oligosaccharide in the unknown sample. One skilled in the art will appreciate that many immunological reagents and methods can be modified to determine the oligosaccharides of this invention without deviating from the scope and spirit of the invention.

### EXAMPLE 2

**Characterization of oligosaccharides:** Oligosaccharides isolated from the urine of a patient having MPS I and MPS II were characterized by their fragmentation patterns as assessed by tandem mass spectrometry (MS/MS). Although mass spectrometry is used in this example, it is to be understood that other quantitative methods, including chromatographic methods, immunoassay and liquid chromatography-mass spectrometry, and anion exchange and size exclusion chromatography can also be used as quantitative methods to determine the quantity of oligosaccharides within a biological sample.

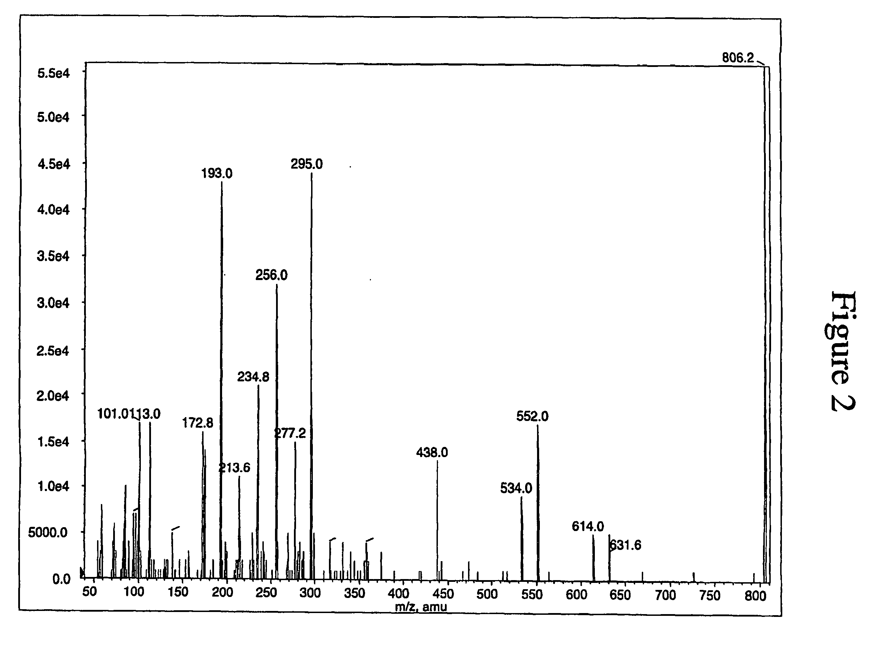

Product ion scans show product ions produced from the parent ion, yielding information about their structure as well as providing suitable ion pairs for MRM. Figure 2 shows the mass spectra of a disaccharide found in MPS I urine. The parent ion mass to charge ratio ("*m*/*z*") is 806.2, representing a [M-H]⁻¹ sulfated disaccharide isolated from MPS I urine, showing an array of daughter ions, including *m*/*z* 256 and 295 used for the MRM pairs.

**Enzymatic cleavage.** Furthermore, the nature of the sugar residues within a particular oligosaccharide can be further characterized by enzymatic cleavage with recombinant enzymes deficient in the particular MPS. Derivatized oligosaccharides (100 µl) were digested with 5ng rhIDUA in 50 mM NH₄ acetate buffer pH 4.0 for 24 hr at 37°C. One tenth of this digest was analyzed by mass spectrometry and the remainder was digested with either 5ng of recombinant N-acetylgalactosamine-4-sulfatase (50 mM ammonium acetate buffer pH 5.6) or 5ng of N-acetylglucosamine-6-sulfatase (50 mM ammonium formate buffer, pH 5.0) for 24 hr at 37°C. These digests were also analyzed by ESI-MS/MS.

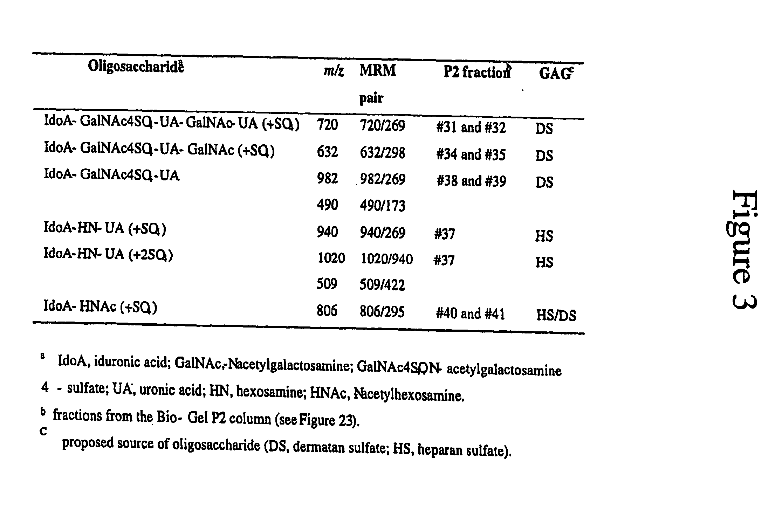

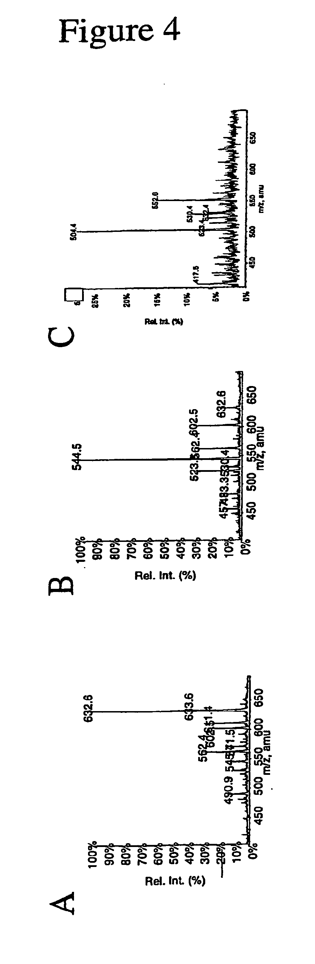

The oligosaccharide structures in fractions #31 to #41 from the Bio-Gel P2 column were characterised by ESI-MSMS to elucidate partial structure and identify a suitable product ion for MRM monitoring Figure 3. Figure 3 shows the oligosaccharides in MPS I urine, wherein penta-, tetra-, tri- and disaccharides were purified from MPS I urine and identified in fractions from a Bio-Gel P2 column according to size. Each oligosaccharide was further characterised by MS/MS and a product ion identified for multiple reaction monitoring ("MRM"). The fragmentation patterns were used to delineate structural composition. None of these oligosaccharides identified in the fractionated MPS I urine were seen by mass spectrometry in the control urine. The control urine contained less than 10 % of the total UA present in the urine from the MPS I patient. Fractions #32, #35, #37, #39 and #40 were treated with rhIDUA to determine whether IdoA was present at the non reducing end. In every instance a loss of 193 amu was seen confirming this terminal residue as IdoA. Following digestion with rhIDUA, fractions #35 and #39 were subsequently treated with recombinant N-acetylgalactosamine-4-sulfatase and N-acetylglucosamine-6-sulfatase. The loss of 80 amu (proposed loss of SO₃) with recombinant N-acetylgalactosamine-4-sulfatase but not with N-acetylglucosamine-6-sulfatase, identified the residue adjacent to the IdoA as N-acetylgalactosamine-4-sulfate. The mass spectrum of the enzyme digest of the tetrasaccharide is shown in Figure 4. Figure 4 shows the mass spectra of tetrasaccharide (*m*/*z* 632) following recombinant enzyme digests. Figure 4A shows the tetrasaccharide with a *m*/*z* 632.6. Figure 4B shows the same oligosaccharide following recombinant α-L-iduronidase digestion. The *m*/*z* 544.5 corresponds to a loss of uronic acid (176 amu). Figure 4C shows the oligosaccharide treated with recombinant α-L-iduronidase and then recombinant N-acetylgalactosamine-4-sulfatase. The *m*/*z* 504.4 corresponds to a proposed loss of sulfate (80 amu).

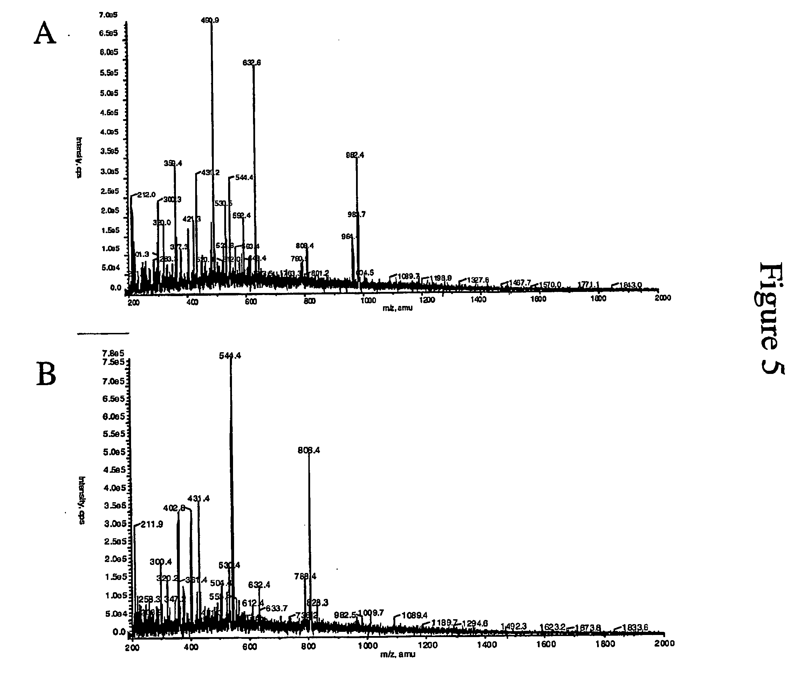

Another example is shown in the mass spectra showing characterization of the non-reducing end of a trisaccharide and a tetrasaccharide isolated from MPS I urine, as shown in Figure 5. A fraction from the Bio-Gel P2 column containing abundant amounts of trisaccharide and tetrasaccharide was subjected to enzymatic digestion with recombinant α-L-iduronidase. The spectra in Figure 5A show the tetrasaccharide [M-H]²⁻ at *m*/*z* 632.6 and a trisaccharide [M-H]⁻ *m*/*z* 982.4 and [M-2H]²⁻ *m*/*z* 490.9 in the absence of α-L-iduronidase. The spectra in Figure 5B show *m*/*z* of each of the ions 632.6, 982.4 and 490.9 following enzymatic digestion with α-L-iduronidase after removal of iduronic acid, producing signals at *m*/*z* 544.4, 806.4 and 402.8 respectively.

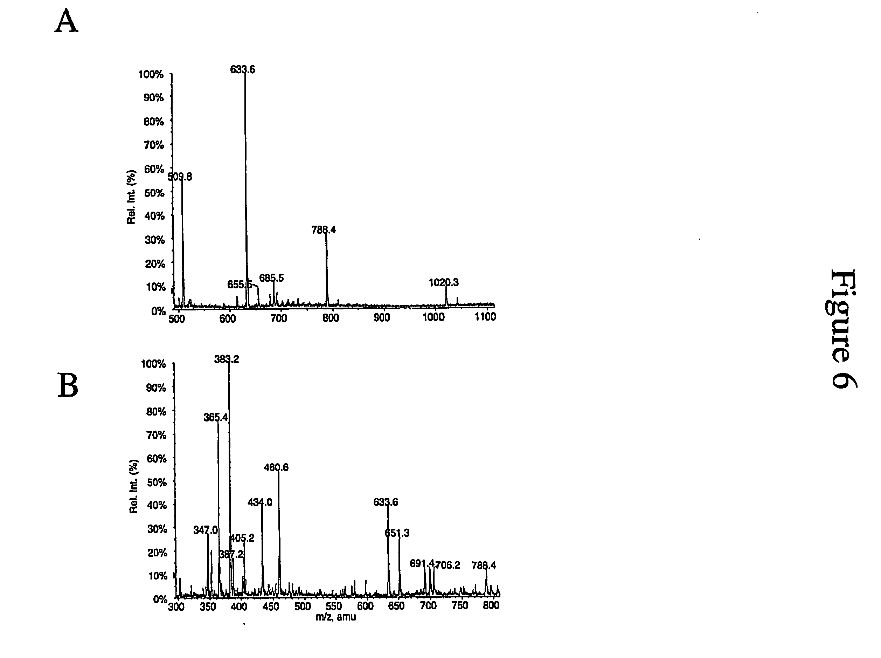

Figure 6 shows a Q1 mass spectra of oligosaccharides purified from urine from a MPS II patient. The oligosaccharides were purified on DEAE Sephacel anion exchange and BioGel P4 size exclusion as described above. Figure 6A shows the Q1 spectra of a trisaccharide fraction with the sugar composition (UA₂, hexosamine ("HN")) the signal at *m*/*z* 509, which corresponds to the [M-2H]² 2S containing species and the signal at *m*/*z* 1020 is the corresponding [M-H]⁻ species. Figure 6B shows the Q1 spectra of a tetrasaccharide fraction with the composition (UA₂, HNAc HN). The signals at *m*/*z* 434 and 460 correspond to the [M-3H]³⁻ 3S, and 4S containing species respectively. The signals at *m*/*z* 651 and 691 result from the corresponding [M-2H]²⁻ species.

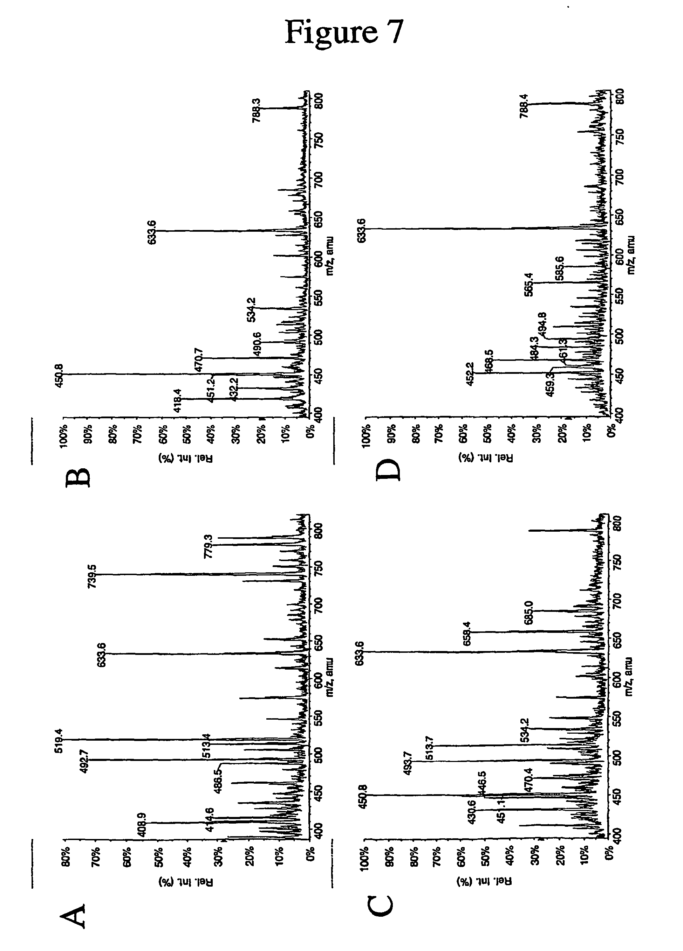

Figure 7 shows a Q1 mass spectra of oligosaccharides purified from urine from a MPS II patient. Oligosaccharides were purified on DEAE Sephacel anion exchange and BioGel P2 size exclusion as described above. Figure 7A shows the Q1 spectra of a pentasaccharide fraction with the composition (UA₃, HNAc, HN) the signals at *m*/*z* 492 and 514 correspond to the [M-3H]³⁻ 3S and 4S containing species respectively and the signals at *m*/*z* 739 and 779 are the corresponding [M-2H]²⁻ species. Figure 7B shows the Q1 spectra of a hexasaccharide fraction with the composition (UA₃, HNAc₃) the signals at *m*/*z* 450, 470 and 490 correspond to the [M-4H]⁴⁻ 4S, 5S and 6S containing species respectively. Figure 7C shows the Q1 spectra of a heptasaccharide fraction with the composition (UA₄, HNAc, HN₂) the signals at *m*/*z* 473, 493 and 513 correspond to the [M-4H]⁴⁻ 4S, 5S and 6S containing species respectively. Figure 7D shows the Q1 spectra of a hexasaccharide fraction with the composition (UA₄, HNAc₄) the signals at *m*/*z* 451, 468 and 484 correspond to the [M-5H]⁵⁻ 5S, 6S and 7S containing species respectively. The signals at *m*/*z* 565 and 585 correspond to the [M-4H]⁴⁻ 5S and 6S containing species.

### EXAMPLE 3

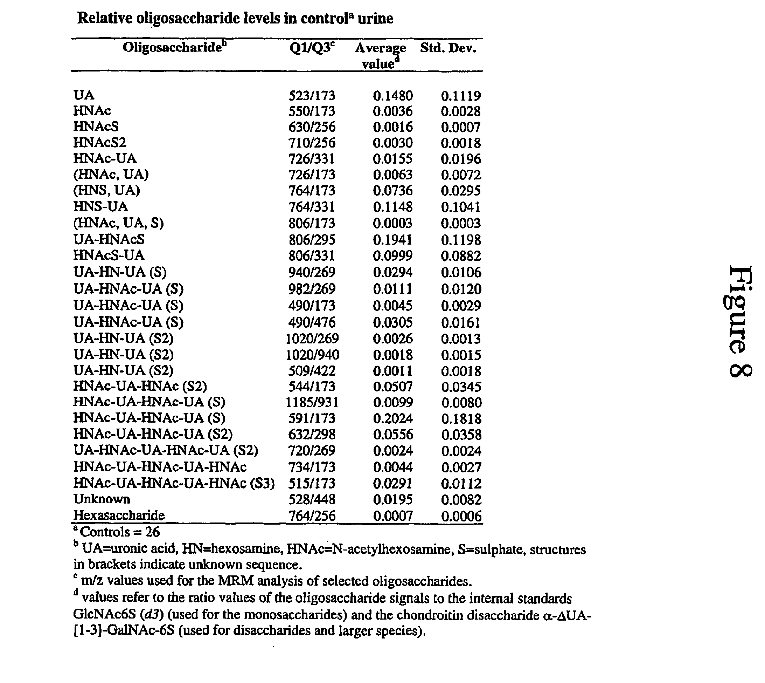

**Identification of oligosaccharides with Mass Spectrometry:** In one embodiment, oligosaccharide markers are identified using electrospray-ionisation tandem mass spectrometry ("ESI/MSMS") and characterized using a combination of enzyme digestion and ESI/MSMS. For example, the determination of oligosaccharide markers by ESI/MSMS enables the identification of MPS affected subjects from the analysis of their body fluids or tissue samples such as urine, plasma, blood, or skin fibroblasts. Figure 8 shows a table wherein oligosaccharide analytes were identified in MPS patients. Oligosaccharides were purified from urine of MPS patients and analyzed by tandem mass spectrometry. Voltages were optimized and MRM ion pairs were identified to enable the rapid analysis of these oligosaccharide species in biological samples.

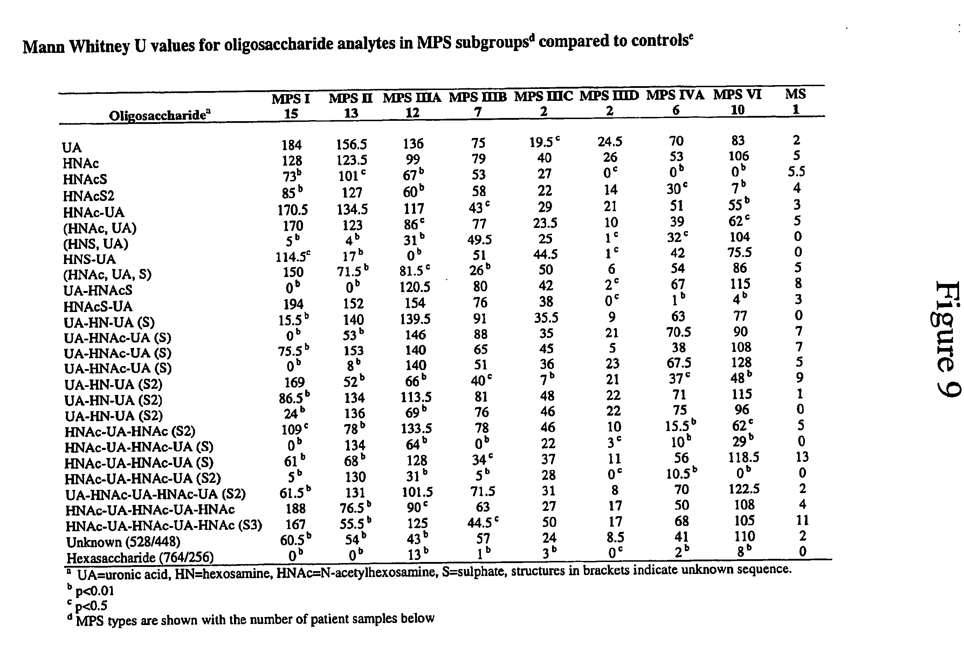

The analysis of urine from control, MPS and multiple sulfatase patients was performed. Urine samples (0.5-1.0 µmol creatinine equivalents) from control (n=26) and MPS and MS affected individuals (70) were derivatized as described above then analyzed on the mass spectrometer for the 27 mono- and oligosaccharides that had been previously identified and characterized from MPS patient urine samples, as shown in Figure 8. The quantification of PMP-derivatized oligosaccharides was performed using the MRM mode. Ion pairs monitored are shown in Figure 8. Each ion pair was monitored for 100 milliseconds using a resolution setting of 1.0 amu at half peak height. For each quantitative measurement, continuous scans were made over the injection period and averaged. Quantification was achieved by relating the peak heights of the PMP-oligosaccharides to the peak height of either the PMP-GlcNAc6S-*d*₃ internal standard for monosaccharides or the PMP-sulfated disaccharide (for all other oligosaccharides). A list of average values and standard deviation ("SD") of the Q1/Q3 for the *m*/*z* values used for the MRM analysis of selected oligosaccharides is shown in Figure 8. Figure 9 shows the Mann Whitney U values for selected oligosaccharide analytes in each MPS subgroup compared to the 26 controls. The number of MPS patients are listed below the MPS type. For each MPS subtype it can be seen that there is a profile of analytes that provide differentiation from the control population.

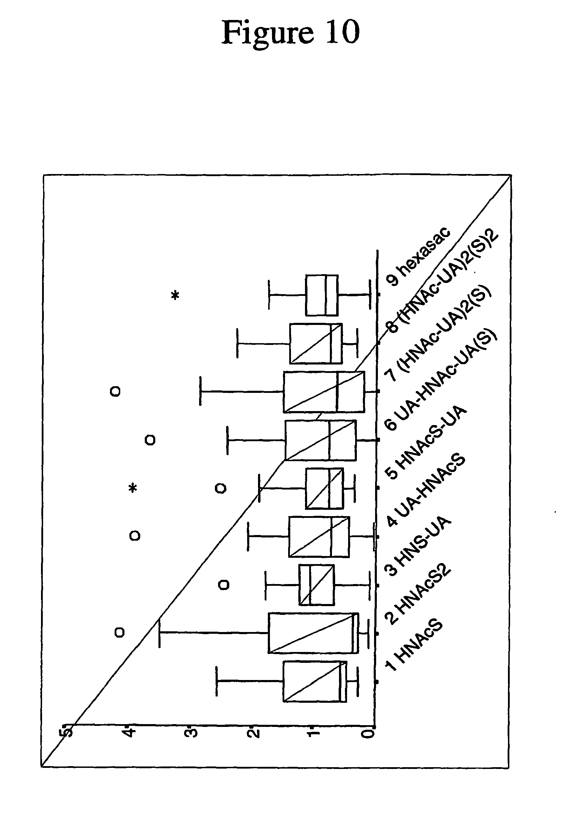

Based on the Mann Whitney U values, nine analytes were selected that best differentiated the MPS and multiple sulfatase groups from the control group. Figure 10 shows a box plot of the relative levels of GAG derived oligosaccharides in control individuals. Urine samples from control individuals (26) were analyzed for GAG derived oligosaccharides as described and the results were normalized, assigning the average value for each analyte an arbitrary value of one. The box plot shows the median level of each analyte in the control group (center bar), the 25th and 75th percentiles (boxes) and the upper and lower limits (upper and lower bars). The circles and stars represent outliers and extreme outliers respectively. If the target quantity in an animal indicates that the MPS disease has progressed, or has not responded to a specific therapy, then a more aggressive therapy may be needed. For example, the dose or length of therapy may be increased. If, on the other hand, the target quantity in an animal indicates that the MPS disease has regressed, or has responded to a specific therapy, then the same of less aggressive therapy may be needed. For example, in a less aggressive therapy, the dose or length of therapy may be decreased.

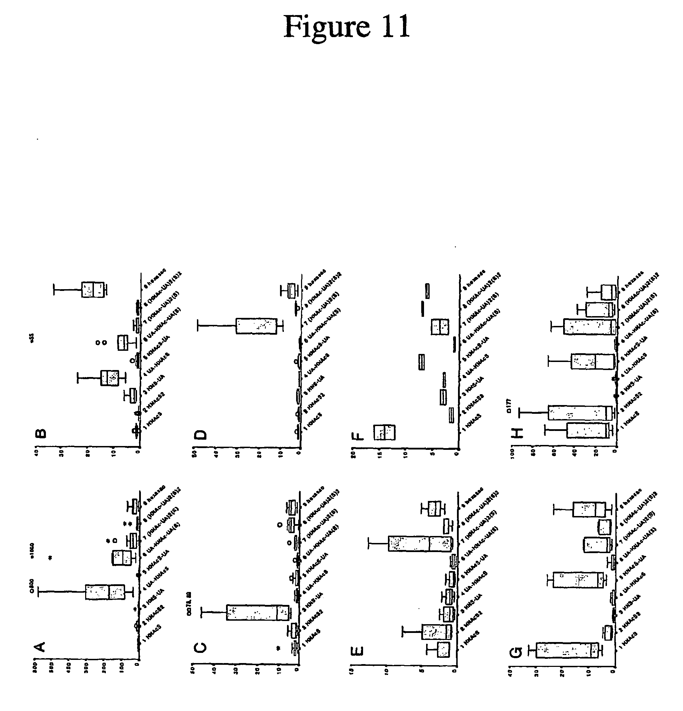

Figure 11 shows the corresponding box plots of the analyte values in each of the MPS groups. There is a characteristic pattern evident for each of the MPS groups that allows for the differentiation of that group from the control group and from the other MPS groups. Urine samples from MPS affected individuals were analyzed for GAG derived oligosaccharides as described and the results were normalized to the control population where the average value for each analyte was assigned a value of one. The box plots shows the results of the selected analytes for MPS I, II, IIIA, IIIB, IIIC, IIID, IVA, VI and multiple sulfatase deficiency (Figure 11 Panels A-H respectively). The plots show the median values (center bars), the 25th and 75th percentiles (boxes) and the upper and lower limits (upper and lower bars). The circles and stars represent outliers and extreme outliers respectively.

Although not wanting to be bound by theory, there are oligosaccharides that relate specifically to the enzyme deficiency in each MPS type and that these are the major species stored in that disorder. The inventors have identified oligosaccharides that are indicative of a specific type of MPS disorder (e.g. MPS-I, MPS-II, etc.) when they are present at elevated levels. Based on these studies a list of predicted storage substrates for each disorder are listed below: in this list the following abbreviations apply, IdoA = iduronic acid; GlcA = glucuronic acid; GalNAc = N-acetylgalactosamine; GlcNAc = N-acetylglucosamine; GlcN = glucosamine; UA = uronic acid; S = sulfate; Gal = galactose.

### MPS I

**Dermatan sulfate fragments :**
IdoA-(GalNAc-(UA-GalNAc)ₙ)(S)ₘ, n=0-5, m=0-11 ;
IdoA-(GalNAc-UA)ₙ(S)ₘ, n=1-6, m=0-12 ;

**Heparan sulfate fragments :**
IdoA-(GlcNAc/GlcN-(UA-GlcNAc/GlcN)ₙ)(S)ₘ, n=0-5, m=0-17 ;
IdoA-(GlcNAc/GlcN-UA)ₙ(S)ₘ, n=1-6, m=0-18.

### MPS II

**Dermatan sulfate fragments:**
IdoA2S-(GalNAc-(UA-GalNAc)ₙ)(S)ₘ, n=0-5, m=0-11;
IdoA2S-(GalNAc-UA)ₙ(S)ₘ, n=1-6, m=0-12.

**Heparan sulfate fragments:**
IdoA2S-(GlcNAc/GlcN-(UA-GlcNAc/GlcN)ₙ)(S)ₘ, n=0-5, m=0-17;
IdoA2S-(GlcNAc/GlcN-UA)ₙ(S)ₘ, n=1-6, m=0-18.

### MPS IIIA

**Heparan sulfate fragments:**
GlcNS-(UA-(GlcNAc/GlcN-UA)ₙ)(S)ₘ, n=0-5, m=0-16;
GlcNS-(UA-GlcNAc/GlcN)ₙ(S)ₘ, n=1-6, m=0-18.

### MPS IIIB

**Heparan sulfate fragments:**
GlcNAc-(UA-(GlcNAc/GlcN-UA)ₙ)(S)ₘ, n=0-5, m=0-16;
GlcNAc-(UA-GlcNAc/GlcN)ₙ(S)ₘ, n=1-6, m=0-18.

### MPS IIIC

**Heparan sulfate fragments:**
GlcN-(UA-(GlcNAc/GlcN-UA)ₙ)(S)ₘ, n=0-5, m=0-16;
GlcN-(UA-GlcNAc/GlcN)ₙ(S)ₘ, n=1-6, m=0-18.

### MPS IIID

**Heparan sulfate fragments**
GlcNAc6S/GlcN6S-(UA-(GlcNAc/GlcN-UA)ₙ)(S)ₘ, n=0-5, m=0-16
GlcNAc6S/GlcN6S-(UA-GlcNAc/GlcN)ₙ(S)ₘ, n=0-6, m=0-18

### MPS IVA

**Keratan sulfate fragments :**
Gal6S-(GlcNAc-(Gal-GlcNAc)ₙ)(S)ₘ, n=0-5, m=0-11;
Gal6S(GlcNAc-Gal)ₙ(S)ₘ, n=0-6, m=0-12.

**Chondroitin sulfate fragments:**
GalNAc6S-(UA-(GalNAc-UA)ₙ)(S)ₘ, n=0-5, m=0-11;
GalNAc6S-(UA-GalNAc)ₙ(S)ₘ, n=0-6, m=0-12.

### MPS IVB

**Keratan sulfate fragments:**
Gal-(GlcNAc-(Gal-GlcNAc)ₙ)(S)ₘ, n=0-5, m=0-11;
Gal-(GlcNAc-Gal)ₙ(S)ₘ, n=1-6, m=0-12.

### MPS VI

**Dermatan sulfate fragments:**
GalNAc4S-(UA-(GalNAc-UA)ₙ)(S)ₘ, n=0-5, m=0-12,
GalNAc4S-(UA-GalNAc)ₙ(S)ₘ, n=0-6, m=0-13.

### MPS VII

**Dermatan sulfate fragments:**
GlcA-GalNAc-(UA-GalNAc)ₙ)(S)ₘ, n=0-5, m=0-11;
GlcA-(GalNAc-UA)ₙ(S)ₘ, n=0-6, m=0-12.

**Heparan sulfate fragments:**
GlcA-(GlcNAcS/GlcNS-(UA-GlcNAc/GlcN)ₙ)(S)ₘ, n=0-5, m=0-17;
GlcA-(GlcNAc/GlcN-UA)ₙ(S)ₘ, n=0-6, m=0-18.

This list covers oligosaccharides up to a degree of polymerization of 12 or 13 sugar residues, and it will be recognised by one skilled in the art that there is a continuum of oligosaccharides from monosaccharides up to the larger species with sizes greater than 5,000 Da. Additionally, one skilled in the art will also recognise that other non-specific oligosaccharides (secondary storage) may be elevated as a result of inhibition of lysosomal function by the primary storage substrate. This list is given as an example.

### EXAMPLE 4

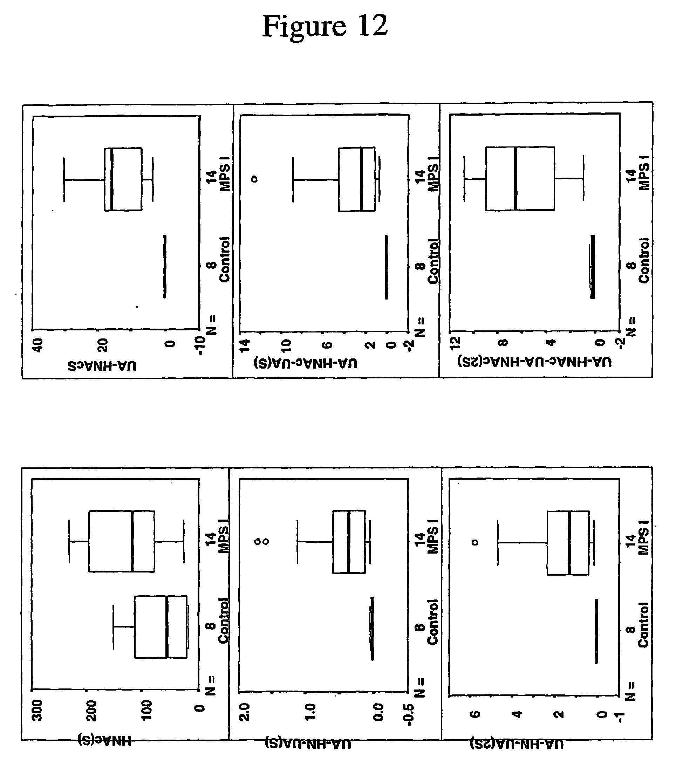

**Diagnosis of the MPS:** As an example of the discriminating power of oligosaccharides isolated from biological samples, the relative level of six oligosaccharides (e.g. HNAc(S), UA-HN-UA(S), UA-HN-UA(2S), UA-HNAcS, UAHNAc-UA(S), and UA-HNAc-UA-HNAc(2S)) in urine from 8 controls and 14 MPS I patients was determined. For 5 of the 6 oligosaccharides measured, there was no overlap between the control and the MPS populations. The relative concentrations of oligosaccharides in urine from control and MPS I patients are shown in Figure 12, and relative quantification was achieved by reference to an internal standard (GlcNAc6S(*d3*)), as described above. Urine samples having a 1 µmol creatinine equivalent from controls and MPS I patients were derivatized and analyzed on the tandem mass spectrometer. Each plot in Figure 12 represents a different oligosaccharide and N = represents the number of samples in each group (i.e. Control or MPS-I). The center horizontal bars show the median value for each group, white boxed areas show the 25^{th} to 75^{th} percentiles. The top and bottom error bars show the limits of the range, and the symbol ("o") represents statistical outliers.

### EXAMPLE 5

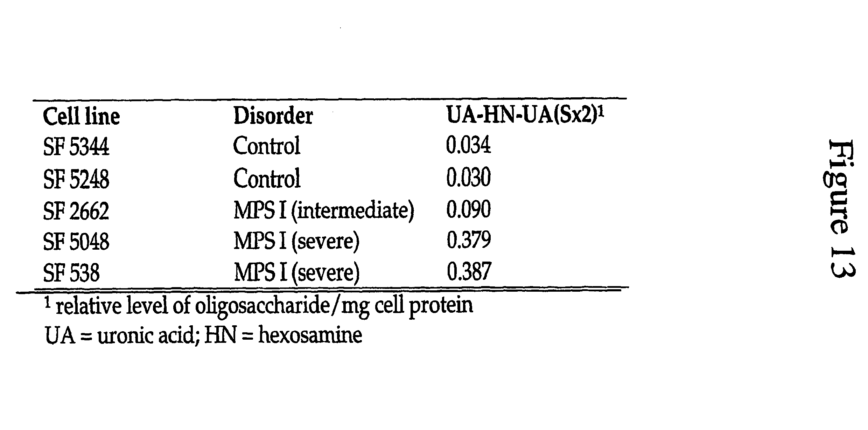

**Determination of phenotype I:** Human skin fibroblasts from 2 controls and 3 MPS I patients were cultured for 6 weeks post-confluence in basel modified eagles (BME) media supplemented with 10 % FCS. Cells were then harvested and cell extracts prepared and subsequently analyzed by mass spectrometry for an oligosaccharide identified in the MPS I urine (Figure 13). A marked difference was observed in the relative levels of the oligosaccharide isolated from cell lines of control and patients with intermediate MPS I. For example, the relative level of UA-HN-UA(2S)/mg cell protein was 0.034 in cell line SF5344 from a control patient, wherein the cell line SF 2662 from a patient having intermediate MPS-I had a relative level of UA-HN-UA(2S)/mg cell protein that was over 3 times higher (e.g. 0.090). A 4+ fold difference between the oligosaccharide level in the two severe patients and the intermediate patient was also evident, which demonstrates a correlative relationship between oligosaccharide level and patient severity.

### EXAMPLE 6

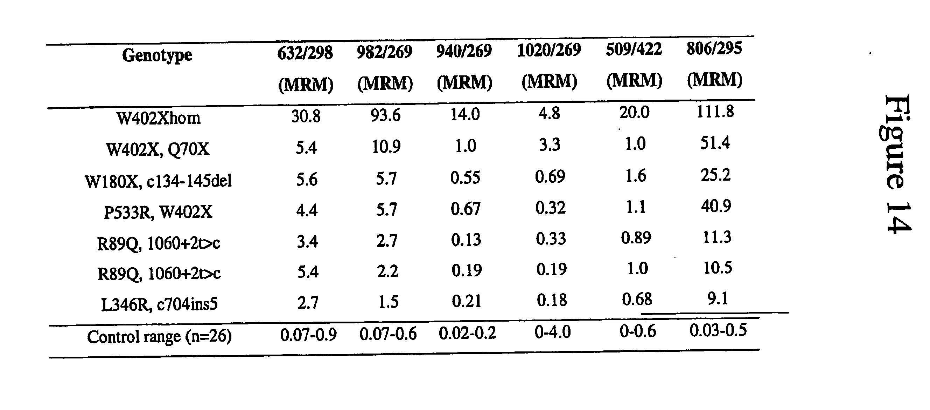

**Determination of Phenotype II.** Relative oligosaccharide levels in urine samples from some MPS I patients also correlated well with genotype. For each urine sample, 0.5 µmol creatinine equivalent was derivatized with PMP and analyzed by mass spectrometry for selected oligosaccharides from Figure 8. Urine samples were collected from MPS I individuals (n=7) and age matched controls (n=26). The urine samples were analyzed by mass spectrometry for selected oligosaccharides. These urine samples were taken from MPS I patients having a range of clinical phenotypes, wherein the genotype and clinical information were documented. As shown in Figure 14, the levels of each MRM pair of selected oligosaccharides was elevated in MPS I patients when compared to the control average.

Molecular genetic analysis does not often give informative prediction of disease severity, but knowing the severity of the disease is paramount for the proper management of MPS I. As shown in Figure 14, the levels of most of the oligosaccharides correlated well with the severity of the mutation. For example, the R89Q allele is considered to be milder than the W402X allele, which is considered to be severe (Scott *et al*.,1993). Accordingly, the levels of oligosaccharides in patients having the W402X allele were higher when compared to the oligosaccharides levels in patients having the milder R89Q allele, as shown in Figure 14. The synthesis of appropriate internal standards will enable the accurate quantification of specific oligosaccharide products of lysosomal storage, which can then be used for the effective prediction of LSD disease phenotype. Although not wanting to be bound by theory, the same approach using other sources of biological samples such as blood, tissue biopsies, or cultured cells will provide similar correlations.

### EXAMPLE 7

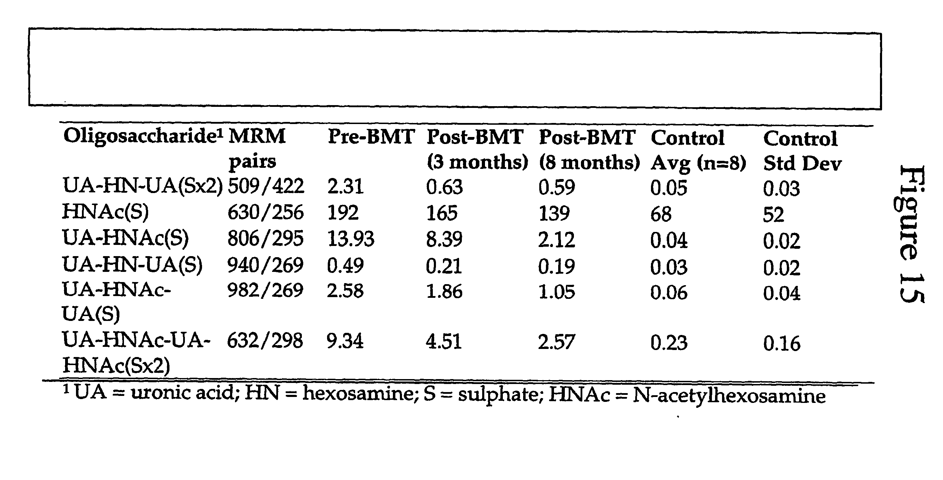

**Monitoring of therapy I**: As an example of how oligosaccharide levels can be used to monitor the effects of therapy, a comparison was conducted in controls and a MPS I patient receiving therapy. Figure 15 shows the relative oligosaccharide levels in a MPS I patient pre- and post- bone marrow transplant ("BMT"). Urine samples were obtained from a MPS-I patient prior to receiving a BMT, and samples were again collected from the patient 3 and 8 months following the BMT. Each urine sample was analyzed for six oligosaccharides that were present in MPS I urine (e.g. UA-HN-UA(2S), HNAc(S), UA-HN-UA(S), UA-HNAc(S), UA-HNAc-UA(S), and UA-HNAc-UA-HNAc(2S)). In all of the oligosaccharides, a continual decrease in relative concentration towards the normal range was observed. For example, the relative urine levels of UA-HNAc(S) in the pre-BMT was 13.93, wherein the relative levels at 3 and 8 months post-BMT were 8.39 and 2.12 respectively. Furthermore, the relative urine levels of UA-HNAc(S) in an average of 8 controls was 0.04.

### EXAMPLE 8

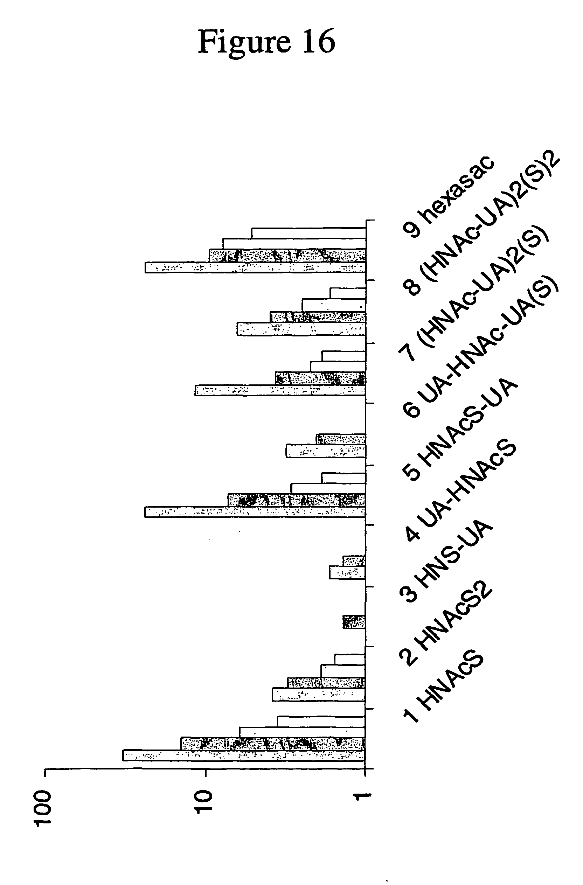

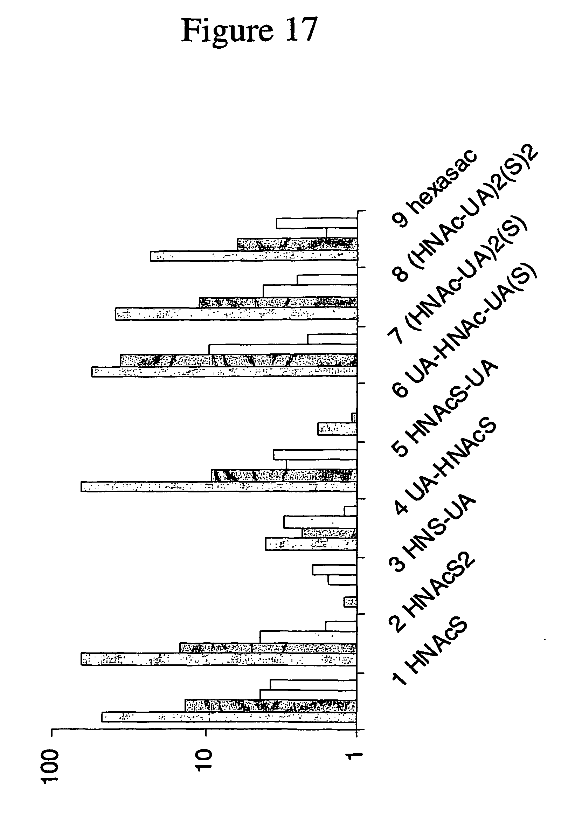

**Monitoring of Therapy II.** Urine samples were collected from a MPS IVA and a MPS VI patient prior to, and at several time points after bone marrow transplantation. Each sample was derivatized and analyzed for selected oligosaccharides. The results (Figure 16 and Figure 17) show a decrease in storage oligosaccharides in these patients corresponding to the length of time post transplant. Figure 16 shows the relative levels of GAG derived oligosaccharides in urine from a MPS IVA affected individual before and after a bone marrow transplant. Urine samples from a MPS IVA affected individuals were collected prior to transplant (solid bar) and at 0.5, 1.6 and 11.6 years post transplant (striped bar, spotted bar and open bar respectively). Each sample was analyzed for GAG derived oligosaccharides as described and the results were normalized to the control population in which the average value for each analyte was assigned a value of one.

Figure 17 shows the relative levels of GAG derived oligosaccharides in urine from a MPS VI affected individual pre- and post-bone marrow transplant. Urine samples from a MPS VI affected individuals were collected prior to transplant (solid bar) and at 1.1, 4.0 and 4.9 years post transplant (striped bar, spotted bar and open bar respectively). Each sample was analyzed for GAG derived oligosaccharides as described and the results were normalized to the control population where the average value for each analyte was assigned a value of one.

### EXAMPLE 9

**Monitoring of therapy III:** A number of animal models of LSD exist, for example, MPS I in dogs, MPS I in cats, MPS VI cats, and MPS VII in mice. The MPS VI disorder in cats has been used as a model for determining MPS and has provided invaluable information regarding both disease pathogenesis and therapy outcomes. For this example, the feline model for MPS type VI was used to demonstrate how MS/MS can measure sulfated mono- and disaccharides for non-invasive, biochemical monitoring during therapy regimes in MPS type VI cats. MPS type VI cats were given a high dose (20 mg/kg) of recombinant feline 4-sulfatase ("rf4S") enzyme replacement therapy for the first month after birth followed by low dose (1 mg/kg) of rf4S for a further 2 months and were compared to animals maintained on 1 mg/kg enzyme replacement therapy for 3 months.

A sulfated monosaccharide N-acetylhexosamine ("HexNAc") and a sulfated disaccharide N-acetylhexosamine-uronic acid ("HexNAc-UA") were elevated in MPS VI cat urine and blood. These markers showed a clear discrimination between the treatment groups during the first four weeks of therapy, values in the low dose group were only slightly lower than the untreated MPS VI cats whereas the high dose group was closer to normal cat values. However, within 2 months of cessation of the high dose therapy there was minimal difference in the oligosaccharide levels with both groups lying between the untreated and unaffected cats. At the completion of the trial, subjective minor improvement was noted in overall physical disease features and also in lysosomal vacuolation in tissues from animals on the initial high-dose enzyme replacement therapy. Initial high dose therapy reduced storage load in the animals but had no lasting clinical benefit over continuous low dose therapy. Although not wanting to be bound by theory, if the target quantity in an animal indicates that the MPS disease has progressed, or has not responded to a specific therapy, then a more aggressive therapy may be needed. For example, the dose or length of therapy may be increased. If, on the other hand, the target quantity in an animal indicates that the MPS disease has regressed, or has responded to a specific therapy, then the same of less aggressive therapy may be needed. For example, in a less aggressive therapy, the dose or length of therapy may be decreased.

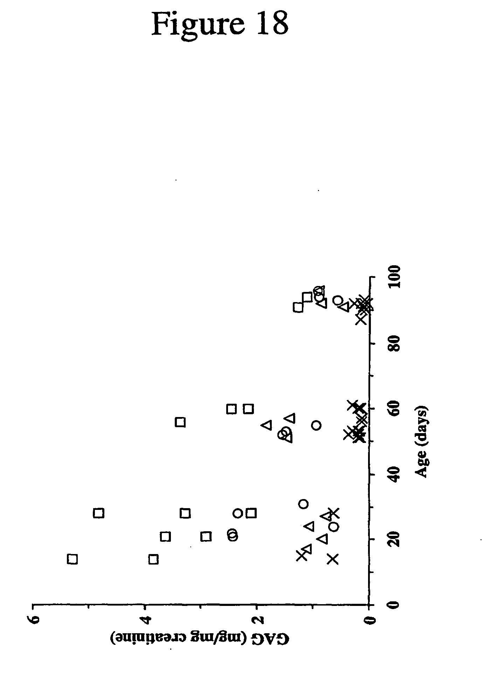

**Monitoring of urinary GAG using the 1,9-dimethylmethylene blue ("DMB") assay.** Total urine GAG as determined by the DMB assay in the low and high dose ERT treated MPS VI cats gave values midway between untreated MPS VI and control animals (Figure 18). GAG levels in high dose treated cats at about 25 days of age were nearly normalized, compared with the low dose treated cats, consistent with the higher rf4S dose rate administered in the first 28 days of age. At days about 55 and 90 the level of urinary GAG was similar in both the high and low dose animals. Figure 18 shows urine collected from normal (x), MPS VI untreated (□), MPS VI low dose treated (○) and MPS VI high dose treated (△) animals at the times indicated. Total urinary GAG was determined using the DMB dye binding assay and normalized to creatinine.

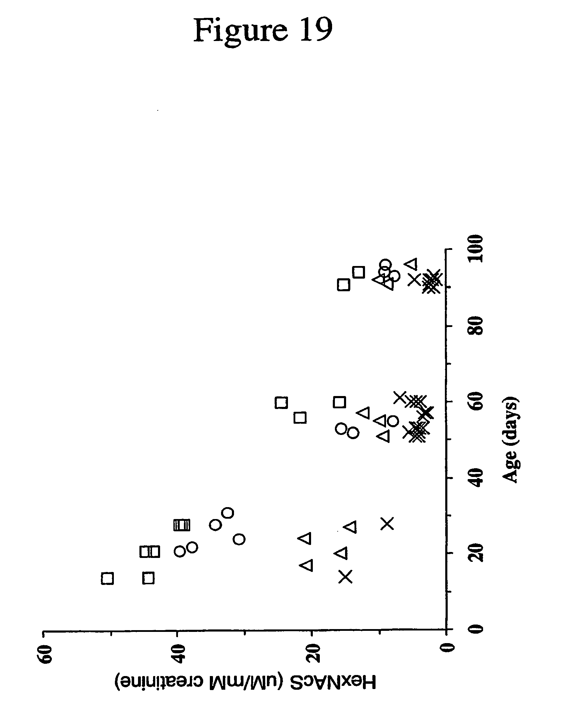

**Monitoring of urinary GAG-derived oligosaccharides using ESI-MS/MS.** Two oligosaccharides previously shown to correlate to disease state in human MPS VI (Ramsay et al 2003 Mol Genet Metab 2003;78(3): 193-204) were analyzed in normal, MPS VI affected and enzyme treated MPS VI cats. Significant levels of the monosaccharide HexNAcS were detected in urine from untreated MPS VI cats. Levels were 4-5x fold higher than in normal animals at 15-25 days of age and 7x the levels found in normal cats at 90 days (Figure 19). Initial monosaccharide sulfate concentrations at 25 days in the low dose treated group were similar to untreated MPS VI cats. The high dose treated group in contrast was much closer to normal levels. However by about 55 days of age (where both groups were on the 1mg/kg dosage), monosaccharide sulfate levels in both ERT dosage groups were similar to each other and approaching levels mid-range between the normal and untreated MPS VI cats. Figure 19 shows urine collected from normal (x), MPS VI untreated (□), MPS VI low dose treated (○) and MPS VI high dose treated (△) animals at the times indicated. The concentration of the monosaccharide HexNAcS was measured using tandem mass spectrometry and normalized to creatinine.

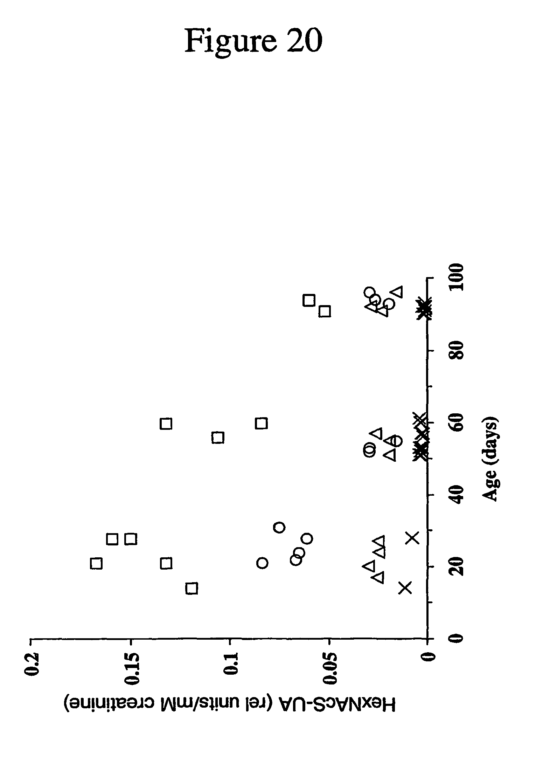

A similar excretion pattern is observed for the sulfated disaccharide HexNAcS-UA, which was about 15x the level observed in normal cats at 15-25 days and about 37x the level observed at 90 days (Figure 20). Commensurate with the monosaccharide sulfate results, the initial amount of sulfated disaccharide in urine of animals in the high dose treated group were closer to normal values whereas sulfated disaccharide amounts in urine of the low dose animals were closer to the untreated MPS VI animals. By about 55 days of age sulfated disaccharide amounts in the urine of both ERT dosage groups were similar to each other.
Figure 20 shows urine collected from normal (x), MPS VI untreated (□), MPS VI low dose treated (○) and MPS VI high dose treated (△) animals at the times indicated. The concentration of the disaccharide HexNAcS-UA was measured using tandem mass spectrometry and normalized to creatinine.

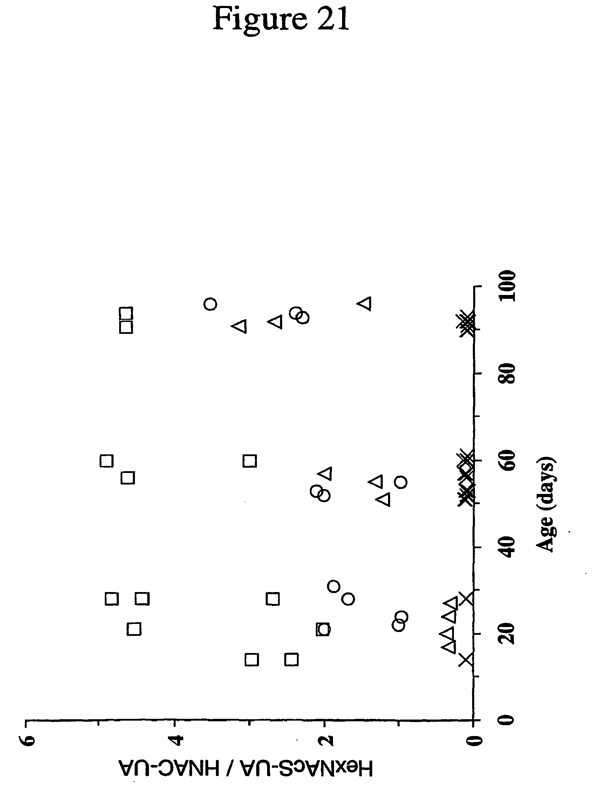

The unsulfated disaccharide HexNAc-UA, represented by the 726/173 MRM pair, is a non-storage GAG-derived analyte that is independent of MPS VI disease state and provides an alternative normative measure to creatinine. When the amount of the sulfated disaccharide HexNAcS-UA is plotted as a ratio of HexNAc-UA a different pattern is observed over time (Figure 21). Figure 21 shows urine collected from normal (x), MPS VI untreated (□), MPS VI low dose treated (○) and MPS VI high dose treated (△) animals at the times indicated. The relative concentrations of the disaccharides HexNAcS-UA and HexNAc-UA were measured using tandem mass spectrometry and are expressed as a ratio.

The ratio of HexNAcS-UA:HexNAc-UA did not decrease with age and instead displayed a slight increase. HexNAcS-UA:HexNAc-UA ratios were about 33x the normal cat value at 21 days compared to 47x the normal cat value at 90 days. At the early time point a difference was noted between animals in the different ERT groups. High dose animals had urinary HexNAcS-UA:HexNAc-UA values close to normal whereas, low dose animals were comparable to untreated MPS VI animals. As with previous results, by about 55 days of therapy the urinary HexNAcS-UA:HexNAc-UA values were similar in both treatment groups. In contrast to previous results normalized to creatinine, the ratio of HexNAcS-UA:HexNAc-UA in the urine of all animals increased with age.

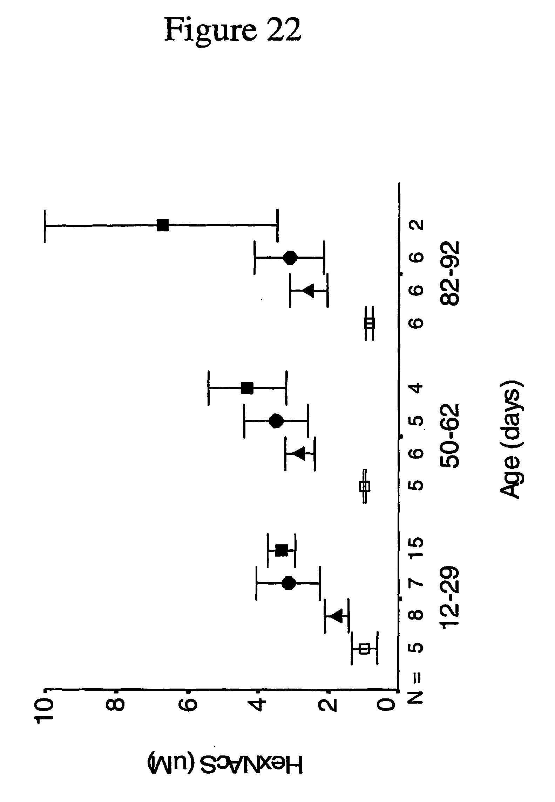

**Monitoring of GAG derived oligosaccharides in blood.** HexNAcS concentration in the untreated MPS VI group displayed an overall increase with age whereas the control cats maintained a consistently low level (Figure 22). Low and high dose ERT cats display dose related levels of HexNAcS in blood. Higher initial levels can be seen in the low dose group compared with the high dose group. Both groups then plateau approximately midway between the untreated MPS VI and normal cats by 90 days of age. A similar pattern in analyte levels in circulation are observed between normal, MPS VI and MPS VI treated animals (Figure 22 compared to Figure 19). However, the discrimination between treatment groups was not as clear as that observed in urine. Although not wanting to be bound by theory, this same approach can be used for other sources of biological samples such as blood, tissue biopsies or cultured cells and will provide similar correlations. Figure 22 shows the concentration of the monosaccharide HexNAcS in blood collected from normal (□), MPS VI untreated (■), MPS VI low dose treated (●) and MPS VI high dose treated (▲) animals. The concentration of the monosaccharide HexNAcS was determined using tandem mass spectrometry. Values are presented as the mean ± 2 std error for the age range indicated. The number of data points per age range is indicated below the x-axis.

### EXAMPLE 10

**Enzyme digestion/chemical digestion of stored oligosaccharides.** Enzyme digestion has been used to characterize stored oligosaccharides in the MPS after purification. An alternate strategy is to use enzyme or chemical digestion to degrade the storage oligosaccharides in a patient sample and thereby provide additional information of the MPS type in addition to improved sensitivity for the quantification of the storage material.

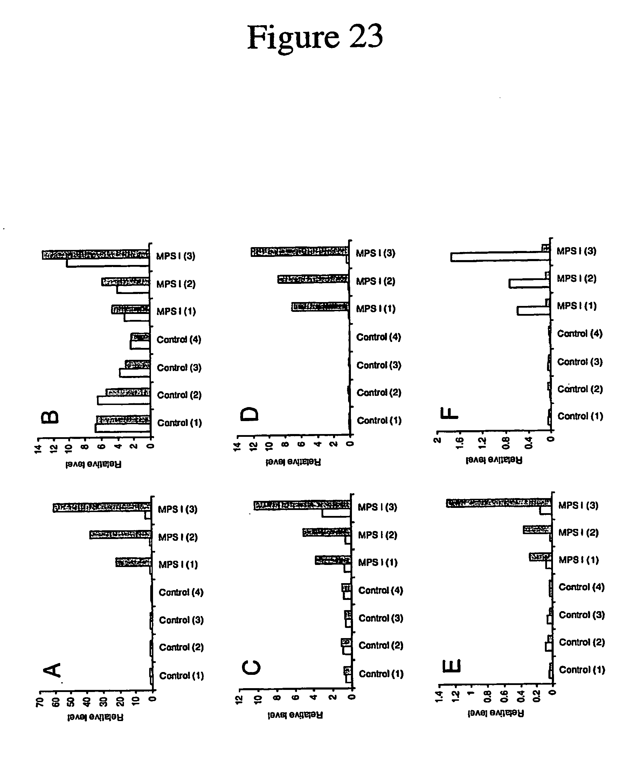

Cultured human skin fibroblasts from control and MPS I affected individuals were grown for 1-8 weeks in culture, then sonicated and the lysate clarified by centrifugation. The lysate was then incubated (100°C, 5 min) to inactivate any lysosomal enzymes prior to digestion with α-L-iduronidase. The oligosaccharides were then digested with α-L-iduronidase, the enzyme deficient in MPS I, and selected oligosaccharides were determined by MSMS before and after enzyme treatment. Figure 23 shows the monosaccharide and oligosaccharide levels in control and MPS I cell lysates before and after treatment with α-L-iduronidase. Cell lysates from control and MPS I affected individuals were digested with α-L-iduronidase and the relative oligosaccharide levels before digestion (open bars) and after digestion (solid bars) were determined. The relative oligosaccharide levels were corrected for cell protein. Figure 23A shows a large increase in the level of uronic acid in the MPS I cell lines but not the control cell lines as a result of the enzyme digestion. The N-acetylglucosamine (Figure 23B) was only slightly increased in the MPS I cell lines and not increased in the control cell lines. Significant increases were also observed in the HNAcS, HNAc-UA and hexosamine-N-sulfate-uronic acid (HNS-UA) oligosaccharides (Figure 23 Panels C, D and E respectively). Corresponding to these increases in oligosaccharides with HNAc at the non-reducing terminus, a decrease in the concentration of UA-HNAcS was observed in the MPS I cell lines but not the control cell lines (Figure 23 F). Figure 23A = UA, Figure 23B = HNAc, Figure 23C = HNAcS, Figure 23D = HNAc-UA, Figure 23E = HNS-UA, Figure 23F = UA-HNAcS. These results demonstrate the value of enzyme digestion of storage substrates to amplify the signal from these substrates and to characterise the particular MPS subtype as only the deficient enzyme would be expected to degrade the primary storage oligosaccharides.

Although not wanting to be bound by theory, enzymes deficient in the other MPS can be used in a similar manner to amplify the signal resulting from the storage of oligosaccharides in patient samples and to characterise the MPS type. Similarly, other GAG degrading endo-enzymes such as the chondroitinases, the heparinases or hyaluronidases could be used to digest the larger GAG derived oligosaccharides to disaccharides, trisaccharides, or tetrasaccharides and thereby amplify the signal. This approach could also release the non-reducing disaccharides, trisaccharides, or tetrasaccharides from each of the larger oligosaccharides, and in the primary storage substrates that is characteristic of a specific MPS disorder.

In addition to endo-enzymes, chemical cleavage could also be used to degrade the larger oligosaccharide species to both amplify the signal and produce disaccharides, trisaccharides or tetrasaccharide species characteristic of the MPS type. For example, nitrous acid digestion of HS will produce disaccharides and other oligosaccharides with anhydro-mannose at the reducing terminus.

### EXAMPLE 11

**LC-MSMS identification of an oligosaccharide in a MPS patient.** GAG was purified from the urine of a MPS VI patient by anion exchange chromatography on a DEAE-Sephacrel column (NaCl elution to 0.1 to 2M) followed by gel filtration on BioGel P10 (200mM, ammonium formate pH 6.0). The fraction containing predominately a hexasaccharide was isolated and used for LC-MSMS sequence determination. For LC-MSMS 0.5µg of uronic acid equivalence of the hexasaccharide was derivatized with O-(4-nitrobenzyl)-hydroxylamine HCl (pNO₂HA) 5mM in 200 mM ammonium formate pH 4.2 buffer incubated at 37°C overnight. Prior to LC-MSMS the material was lyophilized and reconstituted in water containing 0.5mM triethylamine (TEA). The LC was performed on an Alltech C18 column (2mm x 150mm) in buffer A (0.5mM TEA) and buffer B (60% acetonitrile, 0.5mM TEA). The eluant was infused into an API 3000 API-SCIEX tandem mass spectrometer at a rate of 0.2mL/minute. For the enzyme digest 0.5µg UA equivalence was digested with 0.05µg of recombinant 4-sulfatase in ammonium formate buffer pH 4.2 prior to derivatization.

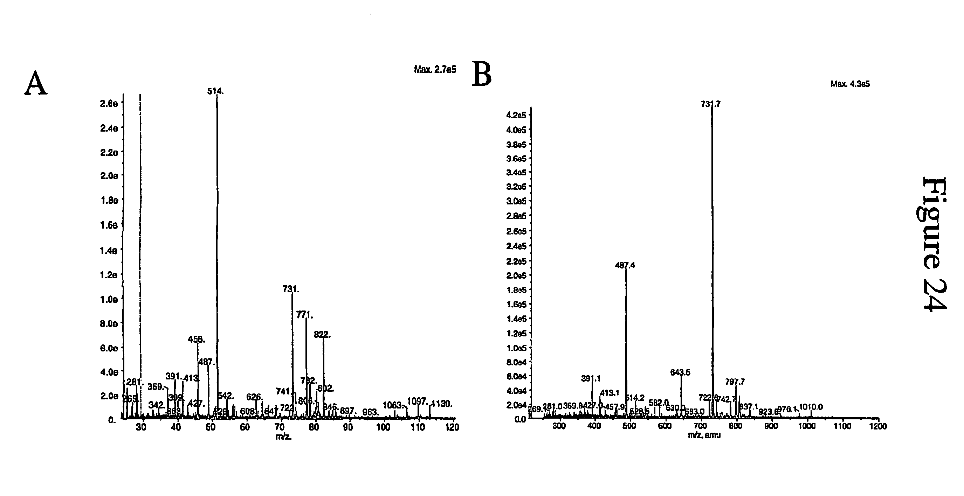

This hexasaccharide was not observed/detected in normal control urine. Figure 24A shows a spectrum that represents the hexasaccharide [M-3H]³⁻ peak at *m*/*z* 514 as the major peak identified as GalNAc4S-UA-GalNAc4S-UA-GalNAc4S-UA-pNo2HA and the peak at 731 represents GalNAc4S-UA-GalNAc-UA-GalNAc4S-UA-pNo2HA. Figure 24B shows the hexasaccharides as shown in Figure 24A after treatment with a recombinant 4-sulfatase. The peak at *m*/*z* 514 has diminished and the major peaks at m/z 487 and *m*/*z* 731 corresponding to the [M-3H]³⁻ and [M-2H]²⁻ disulfated hexasaccharide (GalNAc-UA-GalNAc4S-UA-GalNAc4S-UA-pNo2HA) can be seen.

### EXAMPLE 12

**MPS I phenotype correction in cultured skin fibroblasts.** The catabolism of GAG occurs in two parts, first endoenzymes cleave the polysaccharides to oligosaccharides and then second, an array of exoenzymes sequentially act only at the non reducing end of these oligosaccharides to produce monosaccharides and sulfate. In a lysosomal storage disorder, caused by a deficiency of the exohydrolase α-L-iduronidase, known as MPS I, fragments of two different GAG dermatan and heparan sulfate have been shown to accumulate. Oligosaccharides, penta-, tetra-, tri- and disaccharides that are derived from both GAG, were isolated from the urine of a MPS I patient using a combination of anion-exchange chromatography and gel filtration. These oligosaccharides were identified using electrospray ionisation-tandem mass spectrometry, and shown to have α-L-iduronic acid on their non reducing terminus by their susceptibility to digestion with α-L-iduronidase. Cultured skin fibroblasts from MPS I patients were shown to accumulate the same heparan and dermatan sulfate derived di- and trisaccharides identified in urine with the exception of a dermatan sulfate derived tetrasaccharide. Correction of the MPS I phenotype by supplementation of the culture medium of MPS I fibroblasts with recombinant α-L-iduronidase reduced the level of di- and trisaccharides to that of unaffected control fibroblasts. Furthermore, the level of the dermatan derived tetrasaccharide initially increased and then subsided over time suggesting that it is an intermediate product of catabolism and not as efficiently turned over as a substrate for α-L-iduronidase as other oligosaccharides. These GAG oligosaccharides may prove useful biochemical markers for the clinical severity and management of mucopolysaccharidosis type I. They were shown to be elevated in urine samples from MPS I patients in proportion to their clinical severity, and to decrease in a patient following BMT.

**Experimental protocols.** All cell culture materials were from Life Technologies/Gibco BRL. Recombinant human α-L-iduronidase (rhIDUA), human N-acetylgalactosamine-4-sulfatase and caprine N-acetylglucosamine-6-sulfatase were each prepared from CHO-K1 expression systems. The DEAE Sephacel was from Pharmacia and size exclusion gel Bio-Gel P2 (fine) was from Bio-Rad. The C18 endcapped isolute solid phase extraction cartridges were obtained from International Sorbent Technology and PMP was purchased from Tokyo Kasei Kogyo Co.. All solvents for mass spectrometry were HPLC grade and all other reagents were of analytical grade. Urine samples were submitted to this department for diagnosis and were stored at -20°C.

**Cell culture.** Fibroblasts were cultured from skin biopsies submitted to this hospital for diagnosis. Skin fibroblasts from normal controls and MPS I (intermediate/severe phenotype) patients were cultured in BME containing 10 % (v/v) fetal calf serum (FCS) in 75-cm² culture flasks in a humidified atmosphere containing 5 % CO₂ at 37°C. Post-confluence cell cultures were trypsinised and cells harvested by centrifugation (2000 g). After two washes in phosphate buffered saline, the cell pellet was resuspended in 20 mM TrisHCl, 0.5 M NaCl pH 7, and sonicated for 15 seconds. Total cell protein was determined by the Lowry method. RhIDUA was administered to MPS I and control fibroblasts (six weeks post-confluence), by supplementing the culture medium with 7 µg of the recombinant enzyme/flask. The media (+rhIDUA) was replenished daily for up to five days. MPS I fibroblasts were harvested daily and control fibroblasts were harvested at one and five days.

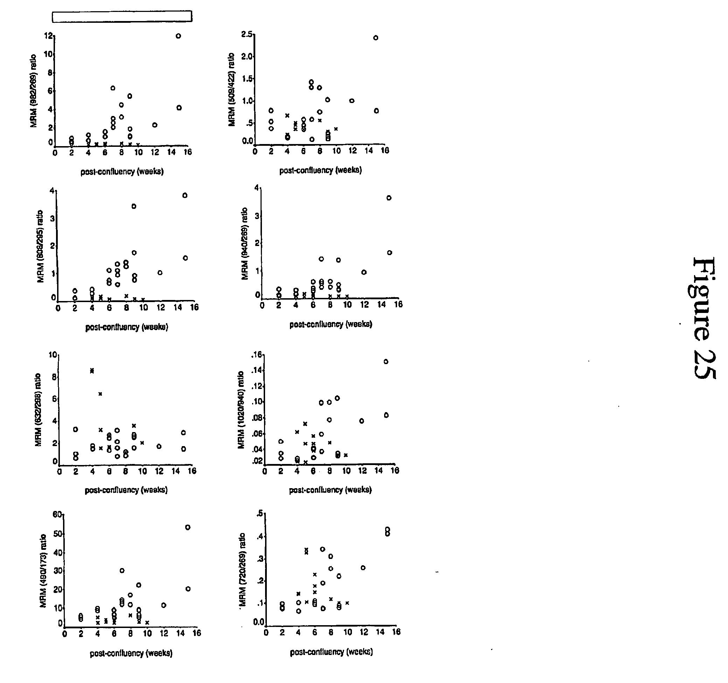

**Storage of oligosaccharides.** A number of confluent cultures of MPS I and control skin fibroblasts were harvested following various lengths of time in culture. Cell extracts were prepared with the internal standard (ISTD), derivatized for mass spectrometry and then analysed for selected MRM pairs. The effect of time in culture (ageing past a state of confluence) on the accumulation of the low molecular weight oligosaccharides is shown in Figure 25. Figure 25 shows the MRM ratios of oligosaccharides in skin fibroblasts post-confluence. A number of MPS I and control skin fibroblasts were cultured 2 to 15 weeks post-confluence. Cell extracts were prepared, derivatized for mass spectrometry and analysed for each MRM pair. The circles and crosses represent skin fibroblasts from MPS I patients and control cell lines respectively. With the exception of the disulfated tetrasaccharide (*m*/*z* 632), all other oligosaccharides accumulated with time in culture in MPS I fibroblasts when compared with the control cell lines. Based on a ratio to the ISTD, the most abundant oligosaccharide identified in skin fibroblasts is the DS derived trisaccharide (*m*/*z* 490/982). This monosulfated trisaccharide is elevated in MPS 1 fibroblasts over control fibroblasts following four weeks in culture. This increase continues with culture time and the level in control skin fibroblasts remains negligible at least for up to 10 weeks in culture. The disaccharide (*m*/*z* 806) is the next most abundant species in the cultured skin fibroblasts with a clear elevation in MPS I cells apparent at 6 weeks post-confluence. The pentasaccharide (*m*/*z* 720) and the HS derived trisaccharides (*m*/*z* 1020/509 and *m*/*z* 940) accumulate in MPS I fibroblasts cultured past their state of confluence.

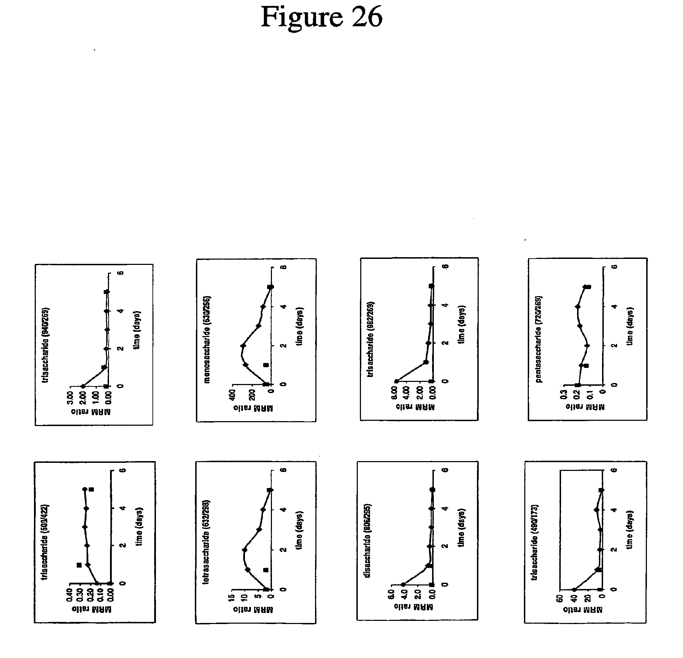

**Correction of Storage.** RhIDUA was administered daily for five days to the culture medium of MPS I fibroblasts and at one and five days in control skin fibroblasts, both of which had been aged for 6 weeks post-confluence. The cells were then harvested and the extracts prepared for mass spectrometry and analysed for each MRM pair. Figure 26 shows the MRM ratios of oligosaccharide levels following recombinant α-L-iduronidase (rhIDUA) addition. MPS I and control skin fibroblasts were maintained in culture six weeks post-confluence. RhIDUA was added to the culture medium of MPS I fibroblasts which, was replenished daily for up to five days. Cells were harvested 24 hr later, derivatized for mass spectrometry and analysed for each MRM pair. Control fibroblasts are shown in the grey boxes, rhIDUA was added daily but cells were harvested at one day and five days only. The 0 time point represents the mean of four values. Figure 26 illustrates a dramatic decrease in the amount of accumulated disaccharide (*mlz* 806) and the trisaccharides (*mlz* 490/982 and *m*/*z* 940) immediately after supplementation of the culture medium with rhIDUA. The levels of pentasaccharide (*m*/*z* 720) and the HS trisaccharide (*m*/*z* 509) did not alter. As seen in Figure 25 both of these oligosaccharides did not accumulate in MPS I fibroblasts after 6 weeks in culture. Interestingly, the amount of tetrasaccharide (*m*/*z* 632) increased sharply in the presence of rhIDUA. Likewise a sulfated monosaccharide (*m*/*z* 630), previously shown to be elevated in some MPS disorders (Hopwood and Elliott, 1985), also increased. This is a sulfated HNAc that is present in urine and fibroblasts but at comparable levels in MPS I and controls.

### EXAMPLE 13

**Materials for a proposed kit.** One aspect of the current invention comprises a kit having all the necessary reagents and materials that are needed for diagnosing a preclinical status, or a clinical status of a MPS disease in a target animal. Generally, a useful kit should comprise: an oligosaccharide derivatization agent used to alter the chemical composition of a target MPS biomarker; an acid solution used to neutralize the derivatization agent following a derivatization reaction; an internal standard used to accurately determine the quantity of the target MPS biomarker; a solid phase extraction column used to purify a derivatized target MPS biomarker; a solid phase extraction column wash solution used to remove impurities from the solid phase extraction column having a bound target MPS biomarker; an oligosaccharide elution solution used to elute the target MPS biomarker from the solid phase extraction column; and a set of instructions for using the kit.

An oligosaccharide derivatization solution comprising 1-phenyl-3methyl-5-pyazolone ("PMP") with NH₃ would be a preferred reagent, however, other derivatizing reagents known in the art are also useful. Similarly, the preferred acid solution comprises formic acid, but some other acids will work equally well. The derivatized oligosaccharide MPS biomarkers are absorbed onto a solid phase extraction column. Although the preferred column for the purification of derivatized oligosaccharides comprises a C18 reverse phase column, other suitable solid phase extraction columns such as an anion exchange or combined phase-C18/anion exchange columns would also work well. Each column in the kit could be packaged as primed or unprimed unit. The preferred column wash solution comprises CHCl₃, but other solutions known in the art will also work. In order to remove the bound oligosaccharides from a bound column an elution solution is needed. Therefore, the preferred kit elution solution comprising CH₃CN and formic acid is used, but other similar solutions will also work.

Also included in the kit is the internal standard, wherein a GlcNAc6S(*d3*) is the preferred internal standard for the determination of GlcNAc6S, while other deuterated standards, with identical structures, would be preferred for other oligosaccharides. Non-physiological oligosaccharides that is similar to the oligosaccharide being investigated can also be use as internal standards. For example, the non-physiological oligosaccharide may be derived from a chondroitinase digestion of chondroitin sulfate having an unsaturated uronic acid at the non-reducing end.

Included on the instruction sheet would be methods describing the process of sample preparation for analysis by mass spectrometry. Additional information would also be useful. For example, sheets that describe the oligosaccharide structures present in, and characteristic of, each MPS disorder type could be included with details of the appropriate mass spectrometer settings to analyze each of the specific MPS biomarker oligosaccharides.

Although mass spectrometry was used in the above examples, it is to be understood that other quantitative methods known in the art, including chromatographic methods, immunoassay and liquid chromatography-mass spectrometry, can also be used as quantitative methods to determine oligosaccharide levels within a biological sample. The information contained herein is useful for the prediction of MPS disease, determining the severity of the MPS disease, monitoring the progression of the MPS disease in patients, and monitoring the progression of therapy.

### REFERENCES

Byers S, Rozaklis T, Brumfield LK, Ranieri E, Hopwood JJ. Glycosaminoglycan accumulation and excretion in the mucopolysaccharidoses: characterization and basis of a diagnostic test for MPS. Mol Genet Metab 1998;65(4):282-90.
Elliott H, Hopwood JJ. Detection of the Sanfilippo D syndrome by the use of a radiolabeled monosaccharide sulfate as the substrate for the estimation of N-acetylglucosamine-6-sulfate sulfatase. Anal Biochem 1984;138(1):205-9.
Fluharty AL, Glick JA, Matusewicz NM, Kihara H. High-performance liquid chromatography determination of unsaturated disaccharides produced from chondroitin sulfates by chondroitinases. Biochem Med 1982;27(3):352-60.
Hopwood JJ, Elliott H. Urinary excretion of sulfated N-acetylhexosamines in patients with various mucopolysaccharidoses. Biochem J 1985;229(3):579-86.
Hua CT, Hopwood JJ, Carlsson SR, Harris RJ, Meikle PJ. Evaluation of the lysosome-associated membrane protein LAMP-2 as a marker for lysosomal storage disorders. Clin Chem 1998;44(10):2094-102.
Kimura A, Hayashi S, Koseki M, Kochi H, Tsurumi K. Fractionation and characterization of urinary heparan sulfate excreted by patients with Sanfilippo syndrome. Tohoku J Exp Med 1984;144(3):227-36.
Kimura A, Hayashi S, Tsurumi K. Chemical structure of urinary dermatan sulfate excreted by a patient with the Hunter syndrome. Tohoku J Exp Med 1980;131(3):241-7.
Klein U, Kresse H, von Figura K. Evidence for degradation of heparan sulfate by endoglycosidases: glucosamine and hexuronic acid are reducing terminals of intracellular heparan sulfate from human skin fibroblasts. Biochem Biophys Res Commun 1976;69(1):158-66.
Klock JC, Starr CM. The different faces of disease. FACE diagnosis of disease. Adv Exp Med Biol 1995;376:13-25.
Kodama C, Ototani N, Isemura M, Yosizawa Z. High-performance liquid chromatography of pyridylamino derivatives of unsaturated disaccharides produced from chondroitin sulfate isomers by chondroitinases. J Biochem (Tokyo) 1984;96(4):1283-7.
Koseki M, Ino S, Kimura A, Tsurumi K. Abnormal urinary excretion in sialoglycoconjugates in patients with mucopolysaccharidosis. Tohoku J Exp Med 1981;135(4):431-9.
Meikle PJ, Brooks DA, Ravenscroft EM, Yan M, Williams RE, Jaunzems AE, et al. Diagnosis of lysosomal storage disorders: evaluation of lysosome-associated membrane protein LAMP-1 as a diagnostic marker. Clin Chem 1997;43(8 Pt 1):1325-35.
Murata K, Murata A, Yoshida K. High-performance liquid chromatographic identification of eight constitutional disaccharides from heparan sulfate isomers digested with heparitinases. J Chromatogr B Biomed Appl 1995;670(1):3-1 0.
Neufeld EF, Meunzer J. The Mucopolysaccharidoses. In: Scriver CR, Beaudet AL, Sly WS, Valle D, editors. The Molecular Basis of Inherited Disease. New York: McGraw-Hill; 2001. p. 3421-52.
Purkiss P, Gibbs DA, Watts RW. Studies on the composition of urinary glycosaminoglycans and oligosaccharides in patients with mucopolysaccharidoses who were receiving fibroblast transplants. Clin Chim Acta 1983;131(1-2):109-21.
Ramsay SL, Meikle PJ, Hopwood JJ. Determination of monosaccharides and disaccharides in mucopolysaccharidoses patients by electrospray ionisation mass spectrometry. Mol Genet Metab 2003;78(3):193-204.
Scott HS, Litjens T, Nelson PV, Thompson PR, Brooks DA, Hopwood JJ, et al. Identification of mutations in the alpha-L-iduronidase gene (IDUA) that cause Hurler and Scheie syndromes. Am J Hum Genet 1993;53(5):973-86.

## Claims

1. A method for diagnosing a pre-clinical status, or a clinical status of a mucopolysaccharidoses ("MPS") disease in a target animal comprising:
(a) determining a target quantity of a target MPS biomarker from a target biological sample taken from the target animal; and
(b) comparing the target quantity to a reference quantity of a reference MPS biomarker;
wherein,
the target MPS biomarker is the same or equivalent to the reference MPS biomarker, and each of the target MPS biomarker and the reference biomarker is an oligosaccharide, wherein the oligosaccharide comprises a sulfated or unsulfated molecule having a sugar length ranging from 1 to 12 residues,
the reference quantity is determined from a reference animal, or group of reference animals, having a known MPS clinical status: and
a deviation of the target quantity of the target MPS biomarker from the reference quantity of the reference MPS biomarker is a pre-clinical or clinical indication of the MPS disease, an indication of a progression of the MPS disease, or an indication of a regression of the MPS disease,
wherein determining the target quantity comprises a mass spectrometric analysis

2. The method of claim 1, wherein the target biological sample or reference biological sample is selected from a cellular extract, blood, plasma, or urine.

3. The method of claim 1 or 2, further comprising derivatizing the target MPS biomarker and the reference MPS biomarker with a derivatizing agent prior to determining the quantity of the target MPS biomarker or the quantity of the reference MPS biomarker.

4. The method of claim 3, wherein the derivatizing agent comprises 1-phenyl-3- methyl-5-pyrazolone ("PMP").

5. The method of any preceding claim wherein the oliogosaccharide comprises a sulfated saccharide molecule having a sugar length ranging from 1 to 12 residues.

6. The method of any of claims 1 to 4 wherein the oligosaccharide identified from the target biological sample comprises a cleavage product of a glycosaminoglycan ("GAG").

7. The method of claim 6 wherein the GAG is heparin sulfate, dermatan sulfate, keratan sulfate, or chondroitin sulfate.

8. The method of any preceding claim wherein the oliogasaccharide is selected from the group consisting of:
(a) a dermatan sulfate fragment that
comprises: IdoA-(GalNAc-(UA-GaINAc)ₙ)(S)ₘ, wherein, n=0-5, m=0-11 ; IdoA - (GaINAc-UA)ₙ(S)ₘ, wherein, n=1-6, m=0-12 ; IdoA2S-(GaINAc-(UA-GalNAc)ₙ)S)ₘ, wherein,
n=0-5, m=0-11; IdoA2S-(GaINAc-UA)ₙ)(S)ₘ, wherein, n=1-6, m=0- 12, GaINAc4S-(UA-(GaINAc-UA)ₙ)(S)ₘ, wherein, n=0-5, m=0-12; GalNAc4S-(UA-GaINAc)ₙ(S)ₘ, wherein, n=0-6, m=0-13; GlcA-GaLNAc-(UA-GalNAc)ₙ)(S)ₘ, wherein, n=0-5, m=0-11; or GIcA-(GaINAc-UA)ₙ(S)ₘ, wherein, n=0-6, m=0-12;
wherein,
IdoA = iduronic acid; GlcA= glucuronic acid; GaINAc = N-acetylgalactosamine; GlcNAc = N-acetylglucosamine; GlcN = glucosamine; UA = uronic acid; S = sulfate; and Gal= galactose;
(b) a heparan sulfate fragment
that comprises: IdoA-(GIcNAc/GIcN-(UA-GIcNAc/GIcN)ₙ)(S)ₘ, wherein n=0-5, m=0-17; IdoA-(GIcNAc/GICN-UA)ₙ(S)ₘ, n = 1-6, m = 0-18; IdoA2S-(GlcNAc/GIcN-(UA-GIcNAc/GIcN)ₙ)(S)ₘ, wherein n=0-5, m=0-17; IdoA2S-(GlcNAc/GIcN-UA)ₙ)(S)ₘ, wherein n=1-6, m=0-18; GIcNS-(UA-(GIcNAc/GIcN-UA)ₙ)(S)ₘ, wherein n=0-5, m=0-16; GlcNS(UA-GlcNAc/GIcN)ₙ(S)ₘ, wherein n=1-6, m=0-18; GIcNAc-(UA-(GIcNAc/GlcN-UA)ₙ)(S)ₘ, wherein n=0-5, m=0-16; GIcNAc-(UA-GIcNAc/GIcN)ₙ(S)ₘ, wherein n=1-6, m=0-18; GIcN-(UA-(GlcNAc/GlcN-UA)ₙ)(S)ₘ, wherein n=0-5, m=0-16; GIcN-(UA-GIcNAc/GIcN)ₙ(S)ₘ, wherein n=1-6, m=0-18; GIcNAc6S/GIcN6S-(UA-(GIcNAc/GIcN-UA)ₙ)(S)ₘ, wherein n=0-5, m=0-16; GIcNAc6S/GIcN6S-(UA-GIcNAc/GIcN)ₙ(S)ₘ, wherein n=0-6, m=0-18; GIcA(GIcNAcS/GIcNS-(UA-GlcNAc/GIcN)ₙ)(S)ₘ, wherein n=0-5, m=0-17 ; or GlcA-(GlcNAc/GlcN-UA)ₙ(S)ₘ, wherein n=0-6, m=0-18;
wherein
IdoA = iduronic acid; GlcA= glucuronic acid; GalNAc = N-acetylgaltactosamine; GlcNAc = N-acetylglucosamime; GlcN= glucosamine; UA= uronic acid; S = sulfate; and gal = galactose;
(c) a keratan sulfate fragment
that comprises: Gal6S-(GlcNAc-(Gal-GlcNAc)ₙ(S)ₘ, wherein n=0-5, m=0-11; Gal6S(GlcNAc-Gal)ₙ(S)ₘ, wherein n=0-6, m= 0-12; Gal-(GlcNAc)ₙ(S)ₘ wherein n=0-5, m=0-11 or Gal-(GlcNAc-Gal)ₙ(S)ₘ, wherein n=1-6, m=0-12;
wherein,
IdoA = iduronic acid; GlcA = glucuronic acid; GalNAc = N-acetylgalactosamine; GlcNAc N-acetylglucosamine; GlcN = glucosamine; UA= uronic acid; S = sulfate; and Gal = galactose ; and (d) a chondroitin sulfate fragment selected from GalNAc6S-(UA-(GalNAc-UA)ₙ)(S)ₘ, wherein n=0-5, m=0-11; or GalNAc6S-(UA-GalNAc)ₙ(S)ₘ, wherein n=0-6, m=0-12;
wherein,
IdoA = iduronic acid; G1cA = glucuronic acid; GaINAc = N-acetylgalactosamine; GlcNAc=
N-acetylglucosamine; GlcN = glucosamine; UA = uronic acid; S = sulfate; and Gal = galactose.

9. The method of claim 1, wherein the mass spectrometry comprises electrospray-ionization tandem mass spectrometry ("ESI-MSMS") or liquid chromatography tandem mass spectrometry ("LC-MSMS").

10. The method of claim 9, wherein the mass spectrometry is carried out in conjunction with an immunoassay, liquid chromatography, anion exchange chromatography, or a combination thereof.

11. The method of any preceding claim wherein the target quantity and the reference quantity are normalized to creatinine or another oligosaccharide.

12. The method of any preceding claim wherein the target animal has received an MPS therapy.

13. The method of any preceding claim wherein the target biological sample and the reference biological sample contain an internal standard.

14. The method of claim 13, wherein the internal standard comprises a deuterated N-acetylglucosamine-6-sulfate ("GlcNAc6S(d3)") or a non physiological oligosaccharide that is similar to the oligosaccharide being investigated.

15. The method of claim 14, wherein the non-physiological oligosaccharide is derived from a chondroitinase digestion of chondroitin sulfate having an unsaturated uronic acid at the non-reducing end.

16. The method of any preceding claim wherein the MPS disease is MPS-I, MPS-II, MPS-IIIA, MPS-IIIB, MPS-VI, MPS-IIIC, MPS-IIID, MPS-IV. or combination thereof.

17. The method of any preceding claim wherein the target animal is a newborn baby.

18. The method of any preceding claim wherein the target MPS biomarker is contacted with an enzyme that characterizes a particular MPS disease subtype,
wherein contacting occurs before determining the target quantity.

19. The method of claim 18, wherein the enzyme comprises α -L-iduronidase.

20. A method as claimed in any preceding claim comprising:
(a) derivatizing a target MPS biomarker with a derivatizing agent forming a derivatized target MPS biomarker;
(b) binding the derivatized target MPS biomarker to an extraction compound to give a bound derivatized target MPS biomarker;
(c) eluting the bound derivatized target MPS biomarker from the extraction compound with an elution solution forming an eluted target MPS biomarker;
(d) determining a target quantity of the dated target MPS biomarker; and
(e) comparing the target quantity with a reference quantity of a reference MPS biomarker;
wherein,
the target MPS biomarker was obtained from a biological sample of a target animal having the MPS biomarker contained therein;
the target MPS biomarker is the same or equivalent to the reference MPS biomarker, and each of the target MPS biomarker and the reference MPS biomarker is an oligosaccharide wherein the oligosaccharide comprises a sulfated or unsulfated molecule having a sugar length ranging from 1 to 12 residues,
the reference quantity is determined in a reference animal, or group of reference animals having a known MPS clinical status; and
a deviation in the quantity of the eluted target MPS biomarker when compared to the reference quantity is a pre-clinical or clinical indication of the MPS disease, a progression of the MPS disease or a regression of the MPS,
wherein determining the target quantity comprises a mass spectrometric analysis.

21. The method of any preceding claim further comprising lyophilizing the target biological sample prior to derivatizing the target MPS biomarker.

22. A kit for diagnosing a pre-clinical status, or a clinical status of a mucopolysaccharidoses ("MPS") disease in a target animal comprising:
(a) an oligosaccharide derivatization agent;
(b) an acid solution;
(c) an internal standard;
(d) a solid phase extraction column;
(e) a solid phase extraction column wash solution; and
(f) an oligosaccharide elution solution,
wherein the oligosaccharide has a length ranging from 1 to 12 residues.

23. The kit of claim 22, wherein the oligosaccharide derivatization agent is a solution comprising 1-phenyl-3-methyl-5-pyrazolone ("PMP").

24. The kit of claim 22 or claim 23, wherein the acid solution is a vial comprising formic acid.

25. The kit of any of claims 23 to 24, wherein the internal standard comprises a deuterated N-acetylglucosamine-6-sulfate ("GlcNAc6S(d3)") or a non-physiological oligosaccharide that is similar to the oligosaccharide being investigated.

26. The kit of claim 25, wherein the non-physiological oligosaccharide is derived from a chondroitase digestion of chondroitin sulfate having an unsaturated uronic acid at the non-reducing end.

27. The kit of any of claims 22 to 26, wherein the solid phase extraction column comprises a C18 reverse phase column.

28. The kit of any of claims 22 to 27 wherein the solid phase extraction column wash solution comprises CHCl₃.

29. The kit of any of claims 22 to 28, wherein the oligosaccharide elution solution comprises CH₃CN and formic acid.

30. A method as discussed in any of claims I to 22 for diagnosing a pre-clinical status, or a clinical status, of a mucopolysaccharidoses ("MPS") disease in a target animal comprising:
(a) determining a target quantity of a target MPS biomarker from a target biological sample taken from the target animal; and
(b) comparing the target quantity to a reference quantity of a reference MPS biomarker;
wherein,
the target MPS biomarker is the same or equivalent to the reference MPS biomarker, and each of the target MPS biomarker and the reference MPS biomarker is an oligosaccharide, and the oligosaccharide is a that comprises: HNAcS; HNAcS₂; HNS-UA; UA-HNAcS; HNAcS-UA; UA-HNAc-UA-S;
(HNAc-UA)₂-S; (HNAc-UA)₂-S₂; or hexasac, wherein, UA = uronic acid; HNAc= N-acetylhexosamine; HN =hexosamine: Hex = hexose: (S) = sulfate not having a sugar residue defined;
the target MPS biomarker and the reference MPS biomarker are derivatized with a derivatizing agent prior to determining the quantity of the target MPS biomarker and the quantity of the reference MPS biomarker, wherein, the derivatizing agent comprises I -phenyl 3-methyl-5-pyrazolone ("PMP");
the target quantity and the reference quantity are normalized to creatinine;
the reference quantity is determined from a reference animal, or group of reference animals, having a known MPS clinical status;
a deviation of the target quantity from the reference quantity is a pre-clinical or clinical indication of the MPS disease, an indication of a progression of the MPS disease, or an indication of a regression of the MPS disease, and the MPS disease is selected from a group comprises: MPS I, MPS II, MPS IIIA, MPS IIIB, MPS IIIC, MPSIIID, MPS IVA, MPS VI, or multiple sulfatase deficiency;
the target quantity and the reference quantity are determined using a mass spectrometry method; and
an internal standard is utilized to accurately determine the target quantity and the reference quantity, wherein the internal standard comprises a deuterated N- acetylglucosamine-6-sulfate ("GIcNAc6S(d3)").

## Patentansprüche

1. Verfahren zur Diagnose eines vorklinischen Status oder eines klinischen Status einer Mucopolysaccharidose-("MPS")-Erkrankung in einem Ziel-Lebewesen, umfassend:
(a) Bestimmen einer Zielmenge eines Ziel-MPS-Biomarkers von einer biologischen Ziel-Probe, die vom Ziel-Lebewesen genommen wurde; und
(b) Vergleichen der Zielmenge mit einer Referenzmenge eines Referenz-MPS-Biomarkers;
wobei
der Ziel-MPS-Biomarker gleich oder äquivalent zum Referenz-MPS-Biomarker ist und der Ziel-MPS-Biomarker und der Referenz-MPS-Biomarker jeweils ein Oligosaccharid sind, wobei das Oligosaccharid ein sulfatiertes oder unsulfatiertes Molekül mit einer Länge des Zuckers im Bereich von 1 bis 12 Resten umfasst,
die Referenzmenge in einem Referenz-Lebewesen oder einer Gruppe von Referenz-Lebewesen mit einem bekannten klinischen MPS-Status bestimmt wird; und
eine Abweichung der Zielmenge des Ziel-MPS-Biomarkers von der Referenzmenge des Referenz-MPS-Biomarkers eine vorklinische oder klinische Indikation der MPS-Erkrankung, eine Indikation eines Fortschreitens der MPS-Erkrankung oder eine Indikation eines Rückgangs der MPS-Erkrankung ist,
wobei das Bestimmen der Zielmenge eine Massenspektromieanalyse umfasst.

2. Verfahren nach Anspruch 1, wobei die biologische Zielprobe oder die biologische Referenzprobe ausgewählt ist aus einem Zellextrakt, Blut, Plasma oder Urin.

3. Verfahren nach Anspruch 1 oder 2, welches ferner die Derivatisierung des Ziel-MPS-Biomarkers und des Referenz-MPS-Biomarkers mit einem derivatisierenden Mittel vor der Bestimmung der Menge des Ziel-MPS-Biomarkers oder der Menge des Referenz-MPS-Biomarkers umfasst.

4. Verfahren nach Anspruch 3, wobei das derivatisierende Mittel 1-Phenyl-3-methyl-5-pyrazolon ("PMP") umfasst.

5. Verfahren nach einem der vorstehenden Ansprüche, wobei das Oligosaccharid ein sulfatiertes Saccharidmolekül mit einer Länge des Zuckers im Bereich von 1 bis 12 Resten umfasst.

6. Verfahren nach einem der Ansprüche 1 bis 4, wobei das durch die biologische Zielprobe ermittelte Oligosaccharid ein Spaltprodukt aus einem Glycosaminoglycan ("GAG") umfasst.

7. Verfahren nach Anspruch 6, wobei das GAG Heparinsulfat, Dermatansulfat, Keratansulfat oder Chondroitinsulfat ist.

8. Verfahren nach einem der vorstehenden Ansprüchen, wobei das Oligosaccharid ausgewählt ist aus der Gruppe bestehend aus:
(a) einem Dermatansulfat-Fragment, das
umfasst: IdoA-(GalNAc-(UA-GalNAc)ₙ)(S)ₘ, wobei n = 0 bis 5, m = 0 bis 11; IdoA-(GalNAc-UA)ₙ(S)ₘ, wobei n = 1 bis 6, m = 0 bis 12; IdoA2S-(GalNAc-(UA-GalNAc)ₙ)(S)ₘ,
wobei
n = 0 bis 5, m = 0 bis 11; IdoA2S-(GalNAc-UA)ₙ)(S)ₘ, wobei n = 1 bis 6, m = 0 bis 12, GalNAc4S-(UA-(GalNAc-UA)ₙ)(S)ₘ, wobei n = 0 bis 5, m = 0 bis 12, GalNAc4S-(UA-GalNAc)ₙ(S)ₘ, wobei n = 0 bis 6, m = 0 bis 13; GlcA-GalNAc-(UA-GalNAc)ₙ)(S)ₘ, wobei n = 0 bis 5, m = 0 bis 11; oder GLcA-(GalNAc-UA)ₙ(S)ₘ, wobei n = 0 bis 6, m = 0 bis 12;
wobei
IdoA = Iduronsäure; GlcA = Glucuronsäure, GalNAc = N-Acetylgalactosamin; GlcNAc = N-Acteylglucosamin; GlcN = Glucosamin; UA = Uronsäure; S = Sulfat; und Gal = Galactose;
(b) einem Heparansulfat-Fragment,
das umfasst: IdoA-(GlcNAc/GlcN-(UA-GlcNAc/GlcN)ₙ)(S)ₘ, wobei n = 0 bis 5, m = 0 bis 17, IdoA-(GlcNAc/GlcN-UA)ₙ(S)ₘ, n = 1 bis 6, m = 0 bis 18, IdoA2S-(GlcNAc/GlcN-(UA-GlcNAc/GlcN)ₙ)(S)ₘ, wobei n = 0 bis 5, m = 0 bis 17, IdoA2S-(GlcNAc/GlcN-UA)ₙ)(S)ₘ, wobei n = 1 bis 6, m = 0 bis 18; GlcNS-(UA-(GlcNAc/GlcN-UA)ₙ)(S)ₘ, wobei n = 0 bis 5, m = 0 bis 16; GlcNS(UA-GlcNAc/GlcN)ₙ(S)ₘ, wobei n = 1 bis 6, m = 0 bis 18; GlcNAc-(UA-(GlcNAc/GlcN-UA)ₙ)(S)ₘ, wobei n = 0 bis 5, m = 0 bis 16; GlcNAc-(UA-GlcNAc/GlcN)ₙ(S)ₘ, wobei n = 1 bis 6, m = 0 bis 18; GlcN-(UA-(GlcNAc/GlcN-UA)ₙ)(S)ₘ, wobei n = 0 bis 5, m = 0 bis 16; GlcN-(UA-GlcNAc/GlcN)ₙ(S)ₘ, wobei n = 1 bis 6, m = 0 bis 18; GlcNAc6S/GlcN6S-(UA-(GlcNAc/GlcN-UA)ₙ)(S)ₘ, wobei n = 0 bis 5, m = 0 bis 16; GlcNAc6S/GlcN6S-(UA-GlcNAc/GlcN)ₙ(S)ₘ, wobei n = 0 bis 6, m = 0 bis 18, GlcA(GlcNAcS/GlcNS-(UA-GlcNAc/GlcN)ₙ)(S)ₘ, wobei n = 0 bis 5 ist, m = 0 bis 17 ist; oder GlcA-(GlcNAc/GlcN-UA)ₙ(S)ₘ, wobei n = 0 bis 6, m = 0 bis 18;
wobei
IdoA = Iduronsäure; GlcA = Glucuronsäure, GalNAc = N-Acetylgalactosamin; GlcNAc = N-Acteylglucosamin; GlcN = Glucosamin; UA = Uronsäure; S = Sulfat; und Gal = Galactose;
(c) einem Keratansulfat-Fragment,
das umfasst: Gal6S(GlcNAc-(Gal-GlcNAc)ₙ(S)ₘ, wobei n = 0 bis 5, m = 0 bis 11; Gal6S(GlcNAc-Gal)ₙ(S)ₘ, wobei n = 0 bis 6, m = 0 bis 12; Gal-(GlcNAc)ₙ(S)ₘ, wobei n = 0 bis 5, m = 0 bis 11 oder Gal-(GlcNAc-Gal)ₙ(S)ₘ, wobei n = 1 bis 6, m = 0 bis 12;
wobei
IdoA = Iduronsäure, GlcA = Glucuronsäure, GalNAc = N-Acetylgalactosamin; GlcNAc = N-Acteylglucosamin; GlcN = Glucosamin; UA = Uronsäure; S = Sulfat; und Gal = Galactose; und
(d) einem Chondroitinsulfat-Fragment, ausgewählt aus GalNAc6S-(UA-(GalNAc-UA)ₙ)(S)ₘ, wobei n = 0 bis 5, m = 0 bis 11; oder GalNAc6S-(UA-GalNAc)ₙ(S)ₘ,
wobei n = 0 bis 6, m = 0 bis 12;
wobei
IdoA = Iduronsäure, GlcA = Glucuronsäure, GalNAc = N-Acetylgalactosamin; GlcNAc = N-Acteylglucosamin; GlcN = Glucosamin; UA = Uronsäure; S = Sulfat; und Gal = Galactose.

9. Verfahren nach Anspruch 1, wobei die Massenspektrometrie Elektrosprühionisierung-Tandemmassenspektrometrie ("ESI-MSMS") oder Flüssigchromatographie-Tandemmassenspektrometrie ("LC-MSMS") umfasst.

10. Verfahren nach Anspruch 9, wobei die Massenspektrometrie in Verbindung mit einem Immuntest, einer Flüssigchromatographie, Anionenaustauschchromatographie oder einer Kombination davon durchgeführt wird.

11. Verfahren nach einem der vorstehenden Ansprüche, wobei die Zielmenge und die Referenzmenge auf Creatinin oder ein anderes Oligosaccharid normalisiert werden.

12. Verfahren nach einem der vorstehenden Ansprüche, wobei das Ziel-Lebewesen eine MPS-Therapie erhalten hat.

13. Verfahren nach einem der vorstehenden Ansprüche, wobei die biologische Zielprobe und die biologische Referenzprobe einen internen Standard enthalten.

14. Verfahren nach Anspruch 13, wobei der interne Standard ein deuteriertes N-Acetylglucosamin-6-sulfat ("GlcNAc6S(d3)") oder ein nicht physiologisches Oligosaccharid, welches dem untersuchten Oligosaccharid ähnlich ist, umfasst.

15. Verfahren nach Anspruch 14, wobei das nicht physiologische Oligosaccharid von einem Chondroitinase-Verdau eines Chondroitinsulfats mit einer ungesättigten Uronsäure am nicht reduzierenden Ende abgeleitet ist.

16. Verfahren nach einem der vorstehenden Ansprüche, wobei die MPS-Erkrankung MPS-I, MPS-II, MPS-IIIA, MPS-IIIB, MPS-VI, MPS-IIIC, MPS-IIID, MPS-IV oder eine Kombination davon ist.

17. Verfahren nach einem der vorstehenden Ansprüche, wobei das Ziel-Lebewesen ein neugeborenes Baby ist.

18. Verfahren nach einem der vorstehenden Ansprüche, wobei der Ziel-MPS-Biomarker mit einem Enzym in Kontakt gebracht wird, das einen speziellen MPS-Erkrankungs-Subtyp darstellt, wobei das Kontaktieren vor der Bestimmung der Zielmenge erfolgt.

19. Verfahren nach Anspruch 18, wobei das Enzym α-L-Iduronidase umfasst.

20. Verfahren nach einem der vorstehenden Ansprüche, umfassend:
(a) Derivatisieren eines Ziel-MPS-Biomarkers mit einem derivatisierenden Mittel, wodurch ein derivatisierter MPS-Biomarker gebildet wird;
(b) Binden des derivatisierten Ziel-MPS-Biomarkers an eine Extraktionsverbindung, um einen gebundenen derivatisierten Ziel-MPS-Biomarker zu erhalten;
(c) Eluieren des gebundenen derivatisierten Ziel-MPS-Biomarkers von der Extraktionsverbindung mit einer Eluierungslösung, wodurch ein eluierter Ziel-MPS-Biomarker gebildet wird;
(d) Bestimmen einer Zielmenge des datierten Ziel-MPS-Biomarkers; und
(e) Vergleichen der Zielmenge mit einer Referenzmenge eines Referenz-MPS-Biomarkers;
wobei
der Ziel-MPS-Biomarker von einer biologischen Probe eines Ziel-Lebewesens mit dem in ihm enthaltenen MPS-Biomarker erhalten wurde;
der Ziel-MPS-Biomarker gleich oder äquivalent zu dem Referenz-MPS-Biomarker ist und der Ziel-MPS-Biomarker und der Referenz-MPS-Biomarker jeweils ein Oligosaccharid sind, wobei das Oligosaccharid ein sulfatiertes oder nicht sulfatiertes Molekül mit einer Länge des Zuckers im Bereich von 1 bis 12 Resten umfasst,
die Referenzmenge in einem Referenz-Lebewesen oder einer Gruppe von Referenz-Lebewesen mit einem bekannten klinischen MPS-Status bestimmt wird; und
eine Abweichung der Menge des eluierten Ziel-MPS-Biomarkers im Vergleich zur Referenzmenge eine vorklinische oder klinische Indikation der MPS-Erkrankung, eines Fortschreitens der MPS-Erkrankung oder eines Rückgangs der MPS-Erkrankung ist,
wobei das Bestimmen der Zielmenge eine Massenspektrometrie-Analyse umfasst.

21. Verfahren nach einem der vorstehenden Ansprüche, ferner umfassend das Lyophilisieren der biologischen Ziel-Probe vor dem Derivatisieren des Ziel-MPS-Biomarkers.

22. Kit für die Diagnose eines vorklinischen Status oder eines klinischen Status einer Mucopolysaccharidose-("MPS")-Erkrankung in einem Ziel-Lebewesen, umfassend:
(a) ein Oligosaccharid-Derivatisierungsmittel;
(b) eine Säurelösung;
(c) einen internen Standard;
(d) eine Festphasen-Extraktionssäule;
(e) eine Festphasen-Extraktionssäulenwaschlösung; und
(f) eine Oligosaccharid-Elutionslösung,
wobei das Oligosaccharid eine Länge im Bereich von 1 bis 12 Resten aufweist.

23. Kit nach Anspruch 22, wobei das Oligosaccharid-Derivatisierungsmittel eine Lösung ist, die 1-Phenyl-3-methyl-5-pyrazolon ("PMP") umfasst.

24. Kit nach Anspruch 22 oder Anspruch 23, wobei die Säurelösung eine Ampulle ist, die Ameisensäure enthält.

25. Kit nach einem der Ansprüche 23 bis 24, wobei der interne Standard ein deuterisiertes N-Acetylglucosamin-6-sulfat ("GlcNAc6S(d3)") oder ein nicht physiologisches Oligosaccharid, welches dem untersuchten Oligosaccharid ähnlich ist, umfasst.

26. Kit nach Anspruch 25, wobei das nicht physiologische Oligosaccharid von einem Chondroitinase-Verdau eines Chondroitinsulfats mit einer ungesättigten Uronsäure am nicht reduzierenden Ende abgeleitet ist.

27. Kit nach einem der Ansprüche 22 bis 26, wobei die Festphasen-Extraktionssäule eine C18-Umkehrphasensäule ist.

28. Kit nach einem der Ansprüche 22 bis 27, wobei die Festphasen-Extraktionssäule-Waschlösung CHCl₃ umfasst.

29. Kit nach einem der Ansprüche 22 bis 28, wobei die Oligosaccharid-Elutionslösung CH₃CN und Ameisensäure umfasst.

30. Verfahren nach einem der Ansprüche 1 bis 22 für die Diagnose eines vorklinischen Status oder eines klinischen Status einer Mucopolysaccharidose-("MPS")-Erkrankung in einem Ziel-Lebewesen, umfassend:
(a) Bestimmen einer Zielmenge eines Ziel-MPS-Biomarkers von einer biologischen Ziel-Probe, die vom Ziel-Lebewesen genommen wurde; und
(b) Vergleichen der Zielmenge mit einer Referenzmenge eines Referenz-MPS-Biomarkers;
wobei
der Ziel-MPS-Biomarker gleich oder äquivalent zu dem Referenz-MPS-Biomarker ist und der Ziel-MPS-Biomarker und der Referenz-MPS-Biomarker jeweils ein Oligosaccharid sind, das HNAcS; HNAcS2; HNS-UA; UA-HNAcS; HNAcS-UA; UA-HNac-UA-S; (HNAc-UA)₂-S; (HNAc-UA)₂(S)₂ oder Hexasac umfasst, wobei UA = Uronsäure; HNAc = N-Acetylhexosamin; HN = Hexosamin; Hex = Hexose; (S) = Sulfat mit keinem definierten Zuckerrest;
der Ziel-MPS-Biomarker und der Referenz-MPS-Biomarker mit einem derivatisierenden Mittel vor der Bestimmung der Menge des Ziel-MPS-Biomarkers oder der Menge des Referenz-MPS-Biomarker derivatisiert werden, wobei das derivatisierende Mittel 1-Phenyl-3-methyl-5-pyrazolon ("PMP") umfasst;
die Zielmenge und die Referenzmenge auf Creatinin normalisiert werden;
die Referenzmenge in einem Referenz-Lebewesen oder einer Gruppe von Referenz-Lebewesen mit einem bekannten klinischen MPS-Status bestimmt wird;
eine Abweichung der Zielmenge von der Referenzmenge eine vorklinische oder klinische Indikation der MPS-Erkrankung, eine Indikation eines Fortschreitens der MPS-Erkrankung oder eine Indikation eines Rückgangs der MPS-Erkrankung ist, und die MPS-Erkrankung ausgewählt ist aus der folgenden Gruppe: MPS I, MPS II, MPS IIIA, MPS IIIB, MPS IIIC, MPS IIID, MPS IVA, MPS VI oder einer multiplen Sulfatasestörung;
die Zielmenge und die Referenzmenge unter Verwendung eines Massenspektrometrie-Verfahrens bestimmt werden; und
ein interner Standard verwendet wird, um die Zielmenge und die Referenzmenge genau zu bestimmen, wobei der interne Standard ein deuteriertes N-Acetylglucosamin-6-sulfat ("GlcNAc6S(d3)") umfasst.

## Revendications

1. Procédé de diagnostic d'un état préclinique, ou d'un état clinique d'une maladie de type mucopolysaccharidoses (« MPS ») chez un animal cible, comprenant les étapes qui consistent à :
(a) déterminer une quantité cible d'un biomarqueur de MPS cible à partir d'un échantillon biologique cible prélevé chez un animal cible ; et à
(b) comparer la quantité cible à une quantité de référence d'un biomarqueur de MPS de référence ;
dans lequel,
le biomarqueur de MPS cible est identique ou équivalent au biomarqueur de MPS de référence, et chacun du biomarqueur de MPS cible et du biomarqueur de référence est un oligosaccharide, l'oligosaccharide comprenant une molécule sulfatée ou non sulfatée ayant une longueur de sucre de 1 à 12 résidus,
la quantité de référence est déterminée à partir d'un animal de référence, ou d'un groupe d'animaux de référence, présentant un état clinique connu en matière de MPS ; et
un écart de la quantité cible du biomarqueur de MPS cible par rapport à la quantité de référence du biomarqueur de MPS de référence est une indication préclinique ou clinique de la maladie MPS, une indication d'une évolution de la maladie MPS, ou une indication d'une régression de la maladie MPS,
dans lequel la détermination de la quantité cible comprend une analyse spectrométrique de masse.

2. Procédé selon la revendication 1, dans lequel l'échantillon biologique cible ou l'échantillon biologique de référence est choisi parmi un extrait cellulaire, du sang, du plasma ou de l'urine.

3. Procédé selon la revendication 1 ou 2, comprenant en outre l'étape qui consiste à former un dérivé du biomarqueur de MPS cible et du biomarqueur de MPS de référence au moyen d'un agent de formation de dérivés avant de déterminer la quantité du biomarqueur de MPS cible ou celle du biomarqueur de MPS de référence.

4. Procédé selon la revendication 3, dans lequel l'agent de formation de dérivés comprend la 1-phényl-3-méthyl-5-pyrazolone (« PMP »).

5. Procédé selon l'une quelconque des revendications précédentes,
dans lequel l'oligosaccharide comprend une molécule de saccharide sulfatée ayant une longueur de sucre de 1 à 12 résidus.

6. Procédé selon l'une quelconque des revendications 1 à 4, dans lequel l'oligosaccharide identifié à partir de l'échantillon biologique cible comprend un produit du clivage d'un glycosaminoglycane (« GAG »).

7. Procédé selon la revendication 6 dans lequel le GAG est de l'héparan-sulfate, du dermatan-sulfate, du kératan-sulfate ou du chondroïtine-sulfate.

8. Procédé selon l'une quelconque des revendications précédentes,
dans lequel l'oligosaccharide est choisi dans le groupe constitué par :
(a) un fragment de dermatan-sulfate qui comprend : IdoA-(GalNAc-(UA-GalNAc)ₙ)(S)ₘ, dans lequel n = 0-5, m = 0-11 ; IdoA-(GalNAc-UA)ₙ(S)ₘ, dans lequel n = 1-6, m = 0-12 ; IdoA2S-(GalNAc-(UA-GalNAc)ₙ)(S)ₘ, dans lequel n = 0-5, m = 0-11 ; IdoA2S-(GalNAc-UA)ₙ)(S)ₘ, dans lequel n = 1-6, m = 0-12 ; GalNAc4S-(UA-(GalNAc-UA)ₙ)(S)ₘ, dans lequel n = 0-5, m = 0-12 ; GalNAc4S-(UA-GalNAc)ₙ(S)ₘ, dans lequel n = 0-6, m = 0-13 ; GlcA-GalNAc-(UA-GalNAc)ₙ)(S)ₘ, dans lequel n = 0-5, m = 0-11 ; ou GlcA-(GalNAc-UA)ₙ(S)ₘ, dans lequel n = 0-6, m = 0-12;
dans lesquels,
IdoA = acide iduronique ; GlcA = acide glucuronique ; GalNAc = N-acétylgalactosamine ; GlcNAc = N-acétylglucosamine ; GlcN = glucosamine ; UA = acide uronique ; S = sulfate ; et Gal = galactose ;
(b) un fragment d'héparan-sulfate qui comprend : IdoA-(GlcNAc/GlcN-(UA-GlcNAc/GlcN)ₙ)(S)ₘ, dans lequel n = 0-5, m = 0-17 ; IdoA-(GlcNAc/GlcN-UA)ₙ(S)ₘ, dans lequel n = 1-6, m = 0-18 ; IdoA2S-(GlcNAc/GlcN-(UA-GlcNAc/GlcN)ₙ)(S)ₘ, dans lequel n = 0-5, m = 0-17 ; IdoA2S-(GlcNAc/GlcN-UA)ₙ)(S)ₘ, dans lequel n = 1-6, m = 0-18 ; GlcNS-(UA-(GlcNAc/GlcN-UA)ₙ)(S)ₘ, dans lequel n = 0-5, m = 0-16 ; GlcNS-(UA-GlcNAc/GlcN)ₙ(S)ₘ, dans lequel n = 1-6, m = 0-18 ; GlcNAc-(UA-(GlcNAc/GlcN-UA)ₙ)(S)ₘ, dans lequel n = 0-5, m = 0-16 ; GlcNAc-(UA-GlcNAc/GlcN)ₙ(S)ₘ, dans lequel n = 1-6, m = 0-18 ; GlcN-(UA-(GlcNAc/GlcN-UA)ₙ)(S)ₘ, dans lequel n = 0-5, m = 0-16 ; GlcN-(UA-GlcNAc/GlcN)ₙ(S)ₘ, dans lequel n = 1-6, m = 0-18 ; GlcNAc6S/GlcN6S-(UA-(GlcNAc/GlcN-UA)ₙ)(S)ₘ, dans lequel n = 0-5, m = 0-16 ; GlcNAc6S/GlcN6S-(UA-GlcNAc/GlcN)ₙ(S)ₘ, dans lequel n = 0-6, m = 0-18 ; GlcA-(GlcNAcS/GlcNS-(UA-GlcNAc/GlcN)ₙ)(S)ₘ, dans lequel n = 0-5, m = 0-17 ; ou GlcA- (GlcNAc/GlcN-UA)ₙ(S)ₘ, dans lequel n = 0-6, m = 0-18 ;
dans lesquels
IdoA = acide iduronique ; GlcA = acide glucuronique ; GalNAc = N-acétylgalactosamine ; GlcNAc = N-acétylglucosamine ; GlcN = glucosamine ; UA = acide uronique ; S = sulfate ; et Gal = galactose ;
(c) un fragment de kératan-sulfate qui comprend : Ga16S-(GlcNAc-(Gal-GlcNAc)ₙ(S)ₘ, dans lequel n = 0-5, m = 0-11 ; Gal6S-(GlcNAc-Gal)ₙ(S)ₘ, dans lequel n = 0-6, m = 0-12 ; Gal-(GlcNAc)ₙ(S)ₘ, dans lequel n = 0-5, m = 0-11 ; ou Gal-(GlcNAc-Gal)ₙ(S)ₘ, dans lequel n = 1-6, m = 0-12 ;
dans lesquels,
IdoA = acide iduronique ; GlcA = acide glucuronique ; GalNAc = N-acétylgalactosamine ; GlcNAc = N-acétylglucosamine ; GlcN = glucosamine ; UA = acide uronique ; S = sulfate ; et Gal = galactose ; et
(d) un fragment de chondroïtine-sulfate choisi parmi GalNAc6S-(UA-(GalNAc-UA)ₙ)(S)ₘ, dans lequel n = 0-5, m = 0-11 ; ou GalNAc6S-(UA-GalNAc)ₙ(S)ₘ, dans lequel, n = 0-6, m = 0-12 ;
dans lesquels
IdoA = acide iduronique ; GlcA = acide glucuronique ; GalNAc = N-acétylgalactosamine ; GlcNAc = N-acétylglucosamine ; GlcN = glucosamine ; UA = acide uronique ; S = sulfate ; et Gal = galactose.

9. Procédé selon la revendication 1, dans lequel la spectrométrie de masse comprend la spectrométrie de masse en tandem à source d'ionisation par électrospray (« ESI-MSMS ») ou la spectrométrie de masse en tandem couplée à la chromatographie en phase liquide (« LC-MSMS »).

10. Procédé selon la revendication 9, dans lequel la spectrométrie de masse est réalisée conjointement avec un dosage immunologique, une chromatographie en phase liquide, une chromatographie échangeuse d'anions ou une combinaison de ceux-ci.

11. Procédé selon l'une quelconque des revendications précédentes,
dans lequel la quantité cible et la quantité de référence sont normalisées par rapport à de la créatinine ou à un autre oligosaccharide.

12. Procédé selon l'une quelconque des revendications précédentes,
dans lequel l'animal cible a reçu un traitement contre la MPS.

13. Procédé selon l'une quelconque des revendications précédentes,
dans lequel l'échantillon biologique cible et l'échantillon biologique de référence contiennent un étalon interne.

14. Procédé selon la revendication 13, dans lequel l'étalon interne comprend un N-acétylglucosamine-6-sulfate déutérié (« GlcNAc6S(d3) ») ou un oligosaccharide non physiologique qui est similaire à l'oligosaccharide que l'on est en train d'étudier.

15. Procédé selon la revendication 14, dans lequel l'oligosaccharide non physiologique est dérivé d'une digestion par chondroïtinase de chondroïtine-sulfate ayant un acide uronique insaturé au niveau de l'extrémité non réductrice.

16. Procédé selon l'une quelconque des revendications précédentes,
dans lequel la maladie MPS est la MPS-I, la MPS-II, la MPS-IIIA, la MPS-IIIB, la MPS-VI, la MPS-IIIC, la MPS-IIID, la MPS-IV, ou une combinaison de celles-ci.

17. Procédé selon l'une quelconque des revendications précédentes,
dans lequel l'animal cible est un petit d'animal nouveau-né.

18. Procédé selon l'une quelconque des revendications précédentes,
dans lequel le biomarqueur de MPS cible est mis en contact avec une enzyme qui caractérise un sous-type de maladie MPS particulier, dans lequel la mise en contact a lieu avant la détermination de la quantité cible.

19. Procédé selon la revendication 18, dans lequel l'enzyme comprend la α-L-iduronidase.

20. Procédé tel que revendiqué dans l'une quelconque des revendications précédentes, comprenant les étapes qui consistent à :
(a) former un dérivé d'un biomarqueur de MPS cible au moyen d'un agent de formation de dérivés formant un dérivé d'un biomarqueur de MPS cible ;
(b) lier le dérivé du biomarqueur de MPS cible à un composé d'extraction afin de donner un dérivé de biomarqueur de MPS cible lié ;
(c) séparer, par élution, le dérivé de biomarqueur de MPS cible lié du composé d'extraction au moyen d'une solution d'élution formant un biomarqueur de MPS cible élué ;
(d) déterminer une quantité cible du biomarqueur de MPS cible élué ; et
(e) comparer la quantité cible à une quantité de référence d'un biomarqueur de MPS de référence ;
dans lequel,
le biomarqueur de MPS cible a été obtenu à partir d'un échantillon biologique d'un animal cible contenant le biomarqueur de MPS ;
le biomarqueur de MPS cible est identique ou équivalent au biomarqueur de MPS de référence, et chacun du biomarqueur de MPS cible et du biomarqueur de MPS de référence est un oligosaccharide, l'oligosaccharide comprenant une molécule sulfatée ou non sulfatée ayant une longueur de sucre de 1 à 12 résidus,
la quantité de référence est déterminée chez un animal de référence, ou chez un groupe d'animaux de référence présentant un état clinique connu en matière de MPS ; et
un écart de la quantité du biomarqueur de MPS cible élué, lorsqu'on la compare à la quantité de référence, est une indication préclinique ou clinique de la maladie MPS, d'une évolution de la maladie MPS ou d'une régression de la MPS,
dans lequel la détermination de la quantité cible comprend une analyse spectrométrique de masse.

21. Procédé selon l'une quelconque des revendications précédentes, comprenant en plus l'étape qui consiste à lyophiliser l'échantillon biologique cible avant de former un dérivé du biomarqueur de MPS cible.

22. Kit de diagnostic d'un état préclinique, ou d'un état clinique d'une maladie de type mucopolysaccharidoses (« MPS ») chez un animal cible, comprenant :
(a) un agent de formation de dérivés oligosaccharidiques ;
(b) une solution d'acide ;
(c) un étalon interne ;
(d) une colonne d'extraction en phase solide ;
(e) une solution de lavage pour colonne d'extraction en phase solide ; et
(f) une solution d'élution d'oligosaccharide,
l'oligosaccharide ayant une longueur allant de 1 à 12 résidus.

23. Kit selon la revendication 22, dans laquelle l'agent de formation de dérivés oligosaccharidiques est une solution comprenant 1-phényl-3-méthyl-5-pyrazolone (« PMP »).

24. Kit selon la revendication 22 ou la revendication 23, dans laquelle la solution d'acide est un flacon comprenant acide formique.

25. Kit selon l'une quelconque des revendications 23 à 24, dans laquelle l'étalon interne comprend un N-acétylglucosamine-6-sulfate déutérié (« GlcNAc6S(d3) ») ou un oligosaccharide non physiologique qui est similaire à l'oligosaccharide que l'on est en train d'étudier.

26. Kit selon la revendication 25, dans laquelle l'oligosaccharide non physiologique est dérivé d'une digestion par chondroïtase de chondroïtine-sulfate ayant un acide uronique insaturé au niveau de l'extrémité non réductrice.

27. Kit selon l'une quelconque des revendications 22 à 26, dans laquelle la colonne d'extraction en phase solide comprend une colonne en phase inverse C18.

28. Kit selon l'une quelconque des revendications 22 à 27, dans laquelle la solution de lavage pour colonne d'extraction en phase solide comprend CHCl₃.

29. Kit selon l'une quelconque des revendications 22 à 28, dans laquelle la solution d'élution d'oligosaccharide comprend CH₃CN et acide formique.

30. Procédé tel que défini dans l'une quelconque des revendications 1 à 22 pour diagnostiquer un état préclinique, ou un état clinque d'une maladie de type mucopolysaccharidoses (« MPS ») chez un animal cible, comprenant les étapes qui consistent à :
(a) déterminer une quantité cible d'un biomarqueur de MPS cible à partir d'un échantillon biologique cible prélevé chez l'animal cible ; et à
(b) comparer la quantité cible à une quantité de référence d'un biomarqueur de MPS de référence ;
dans lequel,
le biomarqueur de MPS cible est identique ou équivalent au biomarqueur de MPS de référence, et chacun du biomarqueur de MPS cible et du biomarqueur de MPS de référence est un oligosaccharide, et l'oligosaccharide est un oligosaccharide qui comprend : HNAcS ; HNAcS2 ; HNS-UA ; UA-HNAcS ; HNAcS-UA ; UA-HNAc-UA-S ; (HNAc-UA)₂-S ; (HNAc-UA)₂(S)₂ ; ou hexasac, dans lesquels, UA = acide uronique ; HNAc = N-acétylhexosamine ; HN = hexosamine ; Hex = hexose ; (S) = sulfate n'ayant pas un résidu de sucre défini ;
le biomarqueur de MPS cible et le biomarqueur de MPS de référence sont transformés en dérivés au moyen d'un agent de formation de dérivés avant de déterminer la quantité du biomarqueur de MPS cible et la quantité du biomarqueur de MPS de référence, l'agent de formation de dérivés comprenant la 1-phényl-3-méthyl-5-pyrazolone (« PMP ») ;
la quantité cible et la quantité de référence sont normalisées par rapport à de la créatinine ;
la quantité de référence est déterminée à partir d'un animal de référence, ou d'un groupe d'animaux de référence, ayant un état clinique connu en matière de MPS ;
un écart de la quantité cible par rapport à la quantité de référence est une indication préclinique ou clinique de la maladie MPS, une indication d'une évolution de la maladie MPS, ou une indication d'une régression de la maladie MPS, et la maladie MPS est choisie dans un groupe qui comprend : la MPS I, la MPS II, la MPS IIIA, la MPS IIIB, la MPS IIIC, la MPS IIID, la MPS IVA, la MPS VI, ou un déficit multiple en sulfatase ;
la quantité cible et la quantité de référence sont déterminées au moyen d'un procédé de spectrométrie de masse ; et
un étalon interne est utilisé pour déterminer exactement la quantité cible et la quantité de référence, l'étalon interne comprenant un N-acétylglucosamine-6-sulfate déutérié (« GlcNAc6S (d3) »).
